# FASCICULE DE BREVET EUROPEEN

(11) **EP 1 268 807 B1**
(45) Date de publication et mention de la délivrance du brevet: **27.09.2006**
(21) Numéro de dépôt: 01919563.5
(22) Date de dépôt: 26.03.2001
(51) Int. Cl.: C12N 15/31, C07K 14/37, G01N 33/68

(54) **GENE 763 DE CHAMPIGNON PHYTOPATOGENE MAGNOPORTHE GRISEA ET SON UTILISATION POUR L'IDENTIFICATION DE COMPOSES FONGICIDES**
GEN 763 AUS DEM PFLANZPATHOGENEN PILZ MAGNOPORTHE GRISEA UND DESSEN VERWENDUNG ZUR NACHWEIS NEUER FUNGIZIDEN
GENE 763 OF PHYTOPATHOGENIC FUNGUS I MAGNAPORTHE GRISEA /I AND USE THEREOF FOR IDENTIFYING FUNGICIDAL COMPOUNDS

(30) Priorité: 31.03.2000 FR 0004101
(43) Date de publication de la demande: 02.01.2003
(73) Titulaire: Bayer CropScience S.A., 69009 Lyon (FR)
(72) Inventeur: LEBRUN, Marc-Henri, F-69002 Lyon (FR); SIBUET, Stéphanie, F-69860 Ouroux (FR); LATORSE, Marie-Pascale, F-69210 Sourcieux-les-Mines (FR)
(74) Mandataire: Tetaz, Franck Claude Edouard
(86) Numéro de dépôt international: PCT/FR2001/000907
(87) Numéro de publication internationale: WO 2001/075115

(56) Documents cités:
- WO-A-99/13094
- DATABASE EMBL [en ligne] EBI; ACC. NO.: AQ449199, 9 avril 1999 (1999-04-09) YU ET AL.: "A BAC end sequencing framework to sequence the Magnaporthe grisea genome" XP002155631
- DATABASE EMBL [en ligne] EBI; ACC. NO.: AQ397193, 15 mars 1999 (1999-03-15) YU ET AL.: "A BAC end sequencing framework to sequence the Magnaporthe grisea genome" XP002155632
- GROSJEAN-COURNOYER M -C ET AL: "MOLECULAR APPROACHES TO ANTIFUNGAL MOLECULE DISCOVERY" SPECIAL PUBLICATION - ROYAL SOCIETY OF CHEMISTRY,GB,ROYAL SOCIETY OF CHEMISTRY, LONDON, vol. 233, 1999, pages 247-255, XP000874586 ISSN: 0260-6291
- LAU ET AL: "Regulatory Genes Controlling MPG1 Expression and Pathogenicity in the Rice Blast Fungus Magnaporthe grisea" PLANT CELL,US,AMERICAN SOCIETY OF PLANT PHYSIOLOGISTS, ROCKVILLE, MD, vol. 8, no. 8, mai 1996 (1996-05), pages 771-781, XP002131415 ISSN: 1040-4651
- POLLEY ET AL.: "Molecular characterisation of meaB, a novel gene affecting nitrogen metabolite repression in Aspergillus nidulans" FEBS LETTERS, vol. 388, no. 2/3, 17 juin 1996 (1996-06-17), pages 200-205, XP002155630 cité dans la demande

### LISTE DE SEQUENCES

<110> Aventis CropScience SA
<120> Gène 763 de champignon phytopathogène et son utilisation pour l'identification de composés fongicides
<130> PM00017 PCT
<140>
   <141>
<150> FR 0004101
   <151> 2000-03-31
<160> 5
<170> PatentIn Ver. 2.1
<210> 1
   <211> 2995
   <212> ADN
   <213> Magnaporthe grisea
<220>
   <221> promoter
   <222> (1)..(705)
<400> 1
   actaccccac cacagagacg cgcttgcagt atttgacgct aaccagaaag ctaggtaccc 60
   aattgccagg ggtcagtaag ccagggttgg cgcaacaagt aggtacgtac ttcccttccc 120
   tgtttgaatt aaaaaaagat taaaaaaaaa aaggtacata gtacataatt acctactttc 180
   catgcttgta ctgtggtaca tgctcgcatc gatctccgat ccactgagtc tggcccccac 240
   cacccctgga ctcttgctgc tgcaagtcct cgacaacctc gtcacgggct tcagtagccc 300
   tgctgctccc ttgatccttg cctgccgggg gagagcgaga gagcccttgg atcatataat 360
   aagctcgaca gaccctccag tgggaccatc agttgcacac atcaagcttg gggccctact 420
   gtatgcctga aatcgaatca aaagacgaga ggtagactac catccaagcg cagatacacc 480
   ttgatcgact ccatagcctc cttctccgat ccatccacca ctcatatcaa tccgtgtgcc 540
   caagcctccc gcttctctcg ctctctccct cttttctctc ttgcatcttc tccttactta 600
   taattcacat cttcactcat cataaccatc tccaaccatg gcaatgtata tgccctcaac 660
   cgcatcttcg acgacacaca ttaggtctcc ctctggaacc cccatagact ctccacaccg 720
   agatgactcg atcaccatcc gccgagccaa gcaagcctgc caagcggaag ggtaagtcat 780
   gacttgggga gctatcttcc ccacgcagag cccacacaac agagtcatta cgcacccagc 840
   tactcggtac ttgggggcat tatgcggctc ttggtcagca gtatctcaac tgacgccatt 900
   gccaattatg ttgtttcaaa ataggtactc gtagcgtctc gactttgacg ccgtcccaac 960
   tggctaggaa gagggcaaac gacagagaag cacagagggc cattcgtgcg cggacaaagg 1020
   aacacatcga acgactcgaa agggaggtcg aagagctcaa gagcaagcag aaccgcgacg 1080
   agaccctcca agaactcatc aggaagaaca agtaccttga gaaggagatt gccaggctgc 1140
   gcgagactta tggcatcccc accccgccaa catctcaccc atatgcgccg tccagtgagt 1200
   gcactctcgc tcatacactg ctttgtaatt ttgccataga attacatggc taaccgggct 1260
   tttctaacac agtctacgac gactctgcag tttccagccg gacatcatca tcttttgggc 1320
   aacattcgcc agactaccat caggtcggag agtatggagc ttcctacatg acaacccccg 1380
   agccttccga accgtggaca tcagtcttac cttgctccaa tgtttcaagc ccagcatcgt 1440
   ccggaagtgc agaagaatac ggatacatcc cgaccagtgt tcccgccgga attgaaggac 1500
   tccctcccac gtcgcgtgtt ggtgcctgca tgaaatatga ggatatggat aacggtgagc 1560
   agtacctcgg tcgcaaataa tattgcaggc atggccacaa aaccctgtgt taaacagcga 1620
   taatcgaacc acgagaccaa gctgacagcg aatgctatgt agaaaacgga tacccccgaa 1680
   gcaacggcgt cccgatgccc ccaacctata tgcaccagca gcagtggccc gtaccataca 1740
   gtgcaaccgt ttactacccc caatcgccgg ctctctgagc ggctaagggt catttggctc 1800
   ccctccgtcg ctggtgagga gccaagtcgg gaccggagat gttaaatagt gttagccgcc 1860
   accgtgtcac gacaatggtc tcacaaaatc aatccggcga cgcccgaaat gggcaaaaac 1920
   ttgaattcaa agagacgaaa gcgaacgacg aattggacga cagagaacat cgaacccgac 1980
   ctccttcgac tcgacccagt catcatttcg gttgatcaag aaattattct agaaaaccca 2040
   aacgccggtt ttcgagacag cattagccaa ttttcttgct cattaacccg agactttgaa 2100
   tgcattagcg tgagggagtg ggccagcggc ggtaacaaac aaaccaggca taactccaac 2160
   gggatttgtc tgttcggatg ttccagcgcg gggacaacat catcaaggcc ttgaacattc 2220
   gaccgatatt cgatcagatc agttcctcac cgccgaactc ccgaagctcg gcacgataaa 2280
   ccctgaagcc attgtcacct gaaaacattc gccttttttt tttgttattc cgatgctcgt 2340
   tcgcaacact acgttttagt tcgacctgtc acacattgcc gccgcgcacg tgtgccacca 2400
   ctagtacagc ttgttaccgg accgaacaac gtttttttcg ccaccgtgcg accctggtcc 2460
   caagtggctt ggtgttgttg cttgcaaaaa aaacctccga caaaccaagt cccccgggct 2520
   ttattagtgt atgtcatcac gcaccccaca agagcctccc gtcactttgc tgtcagcatt 2580
   ttgatggcag gcggggtgac cggcgtgaac ggccaaaagg cgtaggcttt gcgcagtctc 2640
   tccaacaact ttaaagggca attgcgctta gccttggcca gaggtgtctt agctgggata 2700
   aaaaaaacac atattctatt cttatttcgt ttccttgcgc tagcgtactg caacaatttt 2760
   catataattt ttacaccaca atttagggag gttggctgat ttggacggcg gcgtcgtgga 2820
   ggttactcat atatcggcgt atcaagaaag ctcattacca aagctgtatt tgctttttgt 2880
   ttcttgtata tatagactgc attgctcttg tcttgttcat atggagtgga cttgtctttg 2940
   tgtggttgga gtctgggagc atattattgg gtgtgaataa cataggttgg gaatc      2995
<210> 2
   <211> 1969
   <212> ADN
   <213> Magnaporthe grisea
<220>
   <221> CDS
   <222> (17)..(730)
<220>
   <221> 5'UTR
   <222> (1)..(16)
<220>
   <221> 3'UTR
   <222> (731)..(1969)
<400> 2
   agactctcca cacgag atg act cga tca cca tcc gcc gag cca agc aag cct 52
                     Met Thr Arg Ser Pro Ser Ala Glu Pro Ser Lys Pro
                     1               5                   10
   gcc aag cgg aag ggt act cgt agc gtc tcg act ttg acg ccg tcc caa    100
   Ala Lys Arg Lys Gly Thr Arg Ser Val Ser Thr Leu Thr Pro Ser Gln
           15                  20                  25
   ctg gct agg aag agg gca aac gac aga gaa gca cag agg gcc att cgt    148
   Leu Ala Arg Lys Arg Ala Asn Asp Arg Glu Ala Gln Arg Ala Ile Arg
       30                  35                  40
   gcg cgg aca aag gaa cac atc gaa cga ctc gaa agg gag gtc gaa gag    196
   Ala Arg Thr Lys Glu His Ile Glu Arg Leu Glu Arg Glu Val Glu Glu
   45                  50                  55                  60
   ctc aag agc aag cag aac cgc gac gag acc ctc caa gaa ctc atc agg    244
   Leu Lys Ser Lys Gln Asn Arg Asp Glu Thr Leu Gln Glu Leu Ile Arg
                   65                  70                  75
   aag aac aag tac ctt gag aag gag att gcc agg ctg cgc gag act tat    292
   Lys Asn Lys Tyr Leu Glu Lys Glu Ile Ala Arg Leu Arg Glu Thr Tyr
               80                  85                  90
   ggc atc ccc acc ccg cca aca tct cac cca tat gcg ccg tcc atc tac    340
   Gly Ile Pro Thr Pro Pro Thr Ser His Pro Tyr Ala Pro Ser Ile Tyr
               95                  100                 105
   gac gac tct gca gtt tcc agc cgg aca tca tca tct ttt ggg caa cat    388
   Asp Asp Ser Ala Val Ser Ser Arg Thr Ser Ser Ser Phe Gly Gln His
       110                 115                 120
   tcg cca gac tac cat cag gtc gga gag tat gga gct tcc tac atg aca    436
   Ser Pro Asp Tyr His Gln Val Gly Glu Tyr Gly Ala Ser Tyr Met Thr
   125                 130                 135                 140
   acc ccc gag cct tcc gaa ccg tgg aca tca gtc tta cct tgc tcc aat    484
   Thr Pro Glu Pro Ser Glu Pro Trp Thr Ser Val Leu Pro Cys Ser Asn
                   145                 150                 155
   gtt tca agc cca gca tcg tcc gga agt gca gaa gaa tac gga tac atc    532
   Val Ser Ser Pro Ala Ser Ser Gly Ser Ala Glu Glu Tyr Gly Tyr Ile
               160                 165                 170
   ccg acc agt gtt ccc gcc gga att gaa gga ctc cct ccc acg tcg cgt    580
   Pro Thr Ser Val Pro Ala Gly Ile Glu Gly Leu Pro Pro Thr Ser Arg
           175                 180                 185
   gtt ggt gcc tgc atg aaa tat gag gat atg gat aac gaa aac gga tac    628
   Val Gly Ala Cys Met Lys Tyr Glu Asp Met Asp Asn Glu Asn Gly Tyr
       190                 195                 200
   ccc cga agc aac ggc gtc ccg atg ccc cca acc tat atg cac cag cag    676
   Pro Arg Ser Asn Gly Val Pro Met Pro Pro Thr Tyr Met His Gln Gln
   205                 210                 215                 220
   cag tgg ccc gta cca tac agt gca acc gtt tac tac ccc caa tcg ccg    724
   Gln Trp Pro Val Pro Tyr Ser Ala Thr Val Tyr Tyr Pro Gln Ser Pro
                   225                 230                 235
   gct ctc tgagcggcta agggtcattt ggctcccctc cgtcgctggt gaggagccaa     780
   Ala Leu
   gtcgggaccg gagatgttaa atagtgttag ccgccaccgt gtcacgacaa tggtctcaca 840
   aaatcaatcc ggcgacgccc gaaatgggca aaaacttgaa ttcaaagaga cgaaagcgaa 900
   cgacgaattg gacgacagag aacatcgaac ccgacctcct tcgactcgac ccagtcatca 960
   tttcggttga tcaagaaatt attctagaaa acccaaacgc cggttttcga gacagcatta 1020
   gccaattttc ttgctcatta acccgagact ttgaatgcat tagcgtgagg gagtgggcca 1080
   gcggcggtaa caaacaaacc aggcataact ccaacgggat ttgtctgttc ggatgttcca 1140
   gcgcggggac aacatcatca aggccttgaa cattcgaccg atattcgatc agatcagttc 1200
   ctcaccgccg aactcccgaa gctcggcacg ataaaccctg aagccattgt cacctgaaaa 1260
   cattcgcctt ttttttttgt tattccgatg ctcgttcgca acactacgtt ttagttcgac 1320
   ctgtcacaca ttgccgccgc gcacgtgtgc caccactagt acagcttgtt accggaccga 1380
   acaacgtttt tttcgccacc gtgcgaccct ggtcccaagt ggcttggtgt tgttgcttgc 1440
   aaaaaaaacc tccgacaaac caagtccccc gggctttatt agtgtatgtc atcacgcacc 1500
   ccacaagagc ctcccgtcac tttgctgtca gcatcttgat ggcaggcggg gtgaccggcg 1560
   tgaacggcca aaaggcgtag gctttgcgca gtctctccaa caactttaaa gggcaattgc 1620
   gcttagcctt ggccagaggt gtcttagctg ggataaaaaa aacacatatt ctattcttat 1680
   ttcgtttcct tgcgctagcg tactgcaaca attttcatat aatttttaca ccacaattta 1740
   gggaggttgg ctgatttgga cggcggcgtc gtggaggtta ctcatatatc ggcgtatcaa 1800
   gaaagctcat taccaaagct gtatttgctt tttgtttctt gtatatatag actgcattgc 1860
   tcttgtcttg ttcatatgga gtggacttgt ctttgtgtgg ttggagtctg ggagcatatt 1920
   attgggtgtg aataacatag gttgggaatc aaaaaaaaaa aaaaaaaaa             1969
<210> 3
   <211> 238
   <212> PRT
   <213> Magnaporthe grisea
<400> 3
   Met Thr Arg Ser Pro Ser Ala Glu Pro Ser Lys Pro Ala Lys Arg Lys
   1               5                   10                  15
   Gly Thr Arg Ser Val Ser Thr Leu Thr Pro Ser Gln Leu Ala Arg Lys
               20                  25                  30
   Arg Ala Asn Asp Arg Glu Ala Gln Arg Ala Ile Arg Ala Arg Thr Lys
           35                  40                  45
   Glu His Ile Glu Arg Leu Glu Arg Glu Val Glu Glu Leu Lys Ser Lys
       50                  55                  60
   Gln Asn Arg Asp Glu Thr Leu Gln Glu Leu Ile Arg Lys Asn Lys Tyr
   65                  70                  75                  80
   Leu Glu Lys Glu Ile Ala Arg Leu Arg Glu Thr Tyr Gly Ile Pro Thr
                   85                  90                  95
   Pro Pro Thr Ser His Pro Tyr Ala Pro Ser Ile Tyr Asp Asp Ser Ala
               100                 105                 110
   Val Ser Ser Arg Thr Ser Ser Ser Phe Gly Gln His Ser Pro Asp Tyr
           115                 120                 125
   His Gln Val Gly Glu Tyr Gly Ala Ser Tyr Met Thr Thr Pro Glu Pro
       130                 135                 140
   Ser Glu Pro Trp Thr Ser Val Leu Pro Cys Ser Asn Val Ser Ser Pro
   145                 150                 155                 160
   Ala Ser Ser Gly Ser Ala Glu Glu Tyr Gly Tyr Ile Pro Thr Ser Val
                   165                 170                 175
   Pro Ala Gly Ile Glu Gly Leu Pro Pro Thr Ser Arg Val Gly Ala Cys
               180                 185                 190
   Met Lys Tyr Glu Asp Met Asp Asn Glu Asn Gly Tyr Pro Arg Ser Asn
           195                 200                 205
   Gly Val Pro Met Pro Pro Thr Tyr Met His Gln Gln Gln Trp Pro Val
       210                 215                 220
   Pro Tyr Ser Ala Thr Val Tyr Tyr Pro Gln Ser Pro Ala Leu
   225                 230                 235
<210> 4
   <211> 783
   <212> ADN
   <213> Neurospora crassa
<220>
   <221> CDS
   <222> (1)..(783)
<400> 4
   atg gct cgg tct cct cct gct ccc act acc ggg gac aat aag ccg ggt      48
   Met Ala Arg Ser Pro Pro Ala Pro Thr Thr Gly Asp Asn Lys Pro Gly
     1               5                  10                  15
   acg gtg aag agg aaa ggc acg aga agc gtc tct acc ctc acg cct tca      96
   Thr Val Lys Arg Lys Gly Thr Arg Ser Val Ser Thr Leu Thr Pro Ser
                20                  25                  30
   cag cta gct agg aag cga gca aat gat cgg gaa gcg caa cgc gca ata     144
   Gln Leu Ala Arg Lys Arg Ala Asn Asp Arg Glu Ala Gln Arg Ala Ile
            35                  40                  45
   aga gct cgc act aag gag ctc att gaa agg tta caa cgc gaa tta gaa     192
   Arg Ala Arg Thr Lys Glu Leu Ile Glu Arg Leu Gln Arg Glu Leu Glu
        50                  55                  60
   gaa tca agg gga agg gag aac cgc gat gga atg gtt cgt gaa ctg ctt     240
   Glu Ser Arg Gly Arg Glu Asn Arg Asp Gly Met Val Arg Glu Leu Leu
    65                  70                  75                  80
   cag aag aac aag gca ttg gaa cac gaa gta cga gca ttg agg gaa gcc     288
   Gln Lys Asn Lys Ala Leu Glu His Glu Val Arg Ala Leu Arg Glu Ala
                    85                  90                  95
   ctt ggc atc ggg aac cga ccg ttt cct caa tct ggc tac gag gtg gat     336
   Leu Gly Ile Gly Asn Arg Pro Phe Pro Gln Ser Gly Tyr Glu Val Asp
               100                 105                 110
   gga ctg caa act tcg cca tca gca gtt cca ggt cgc ggg gca tct atc     384
   Gly Leu Gln Thr Ser Pro Ser Ala Val Pro Gly Arg Gly Ala Ser Ile
           115                 120                 125
   ccc caa ggc tcc acg gac tat ggt gca ccg aca agc ttt ggg tca tct     432
   Pro Gln Gly Ser Thr Asp Tyr Gly Ala Pro Thr Ser Phe Gly Ser Ser
       130                 135                 140
   tac ctc cct act cct gaa cct tgt gag gca tgg cct ccg gtc gtc cct     480
   Tyr Leu Pro Thr Pro Glu Pro Cys Glu Ala Trp Pro Pro Val Val Pro
   145                 150                 155                 160
   gtc tct tca gtc act gtt tct tca gtg gtt tca agt ccg tcg tca tcc     528
   Val Ser Ser Val Thr Val Ser Ser Val Val Ser Ser Pro Ser Ser Ser
                   165                 170                 175
   acg ggg cat cct gat gag tac gcg gct agc cac gtc ccc aca agc gtc     576
   Thr Gly His Pro Asp Glu Tyr Ala Ala Ser His Val Pro Thr Ser Val
               180                 185                 190
   cct tct tcc ttg atg gac tca tct gta atg ggt caa gct acg gga atc    624
   Pro Ser Ser Leu Met Asp Ser Ser Val Met Gly Gln Ala Thr Gly Ile
           195                 200                 205
   tct tgc ctg gac ggt atg aag gtc aac tac gac gag att gaa gct gac    672
   Ser Cys Leu Asp Gly Met Lys Val Asn Tyr Asp Glu Ile Glu Ala Asp
       210                 215                 220
   cgc gga tac tgt ccg acc agc gtt cca cag ccg caa tcc tct tat ctc    720
   Arg Gly Tyr Cys Pro Thr Ser Val Pro Gln Pro Gln Ser Ser Tyr Leu
   225                 230                 235                 240
   ccc cag cag tcc tgg tcc atg tac ccc aca tca act tac tat cca caa    768
   Pro Gln Gln Ser Trp Ser Met Tyr Pro Thr Ser Thr Tyr Tyr Pro Gln
                   245                 250                 255
   tcg cct acg gtt tga                                                783
   Ser Pro Thr Val
               260
<210> 5
   <211> 260
   <212> PRT
   <213> Neurospora crassa
<400> 5
   Met Ala Arg Ser Pro Pro Ala Pro Thr Thr Gly Asp Asn Lys Pro Gly
     1             5                  10                  15
   Thr Val Lys Arg Lys Gly Thr Arg Ser Val Ser Thr Leu Thr Pro Ser
                20                  25                  30
   Gln Leu Ala Arg Lys Arg Ala Asn Asp Arg Glu Ala Gln Arg Ala Ile
            35                  40                  45
   Arg Ala Arg Thr Lys Glu Leu Ile Glu Arg Leu Gln Arg Glu Leu Glu
        50                  55                  60
   Glu Ser Arg Gly Arg Glu Asn Arg Asp Gly Met Val Arg Glu Leu Leu
    65                  70                  75                  80
   Gln Lys Asn Lys Ala Leu Glu His Glu Val Arg Ala Leu Arg Glu Ala
                    85                  90                  95
   Leu Gly Ile Gly Asn Arg Pro Phe Pro Gln Ser Gly Tyr Glu Val Asp
               100                 105                 110
   Gly Leu Gln Thr Ser Pro Ser Ala Val Pro Gly Arg Gly Ala Ser Ile
           115                 120                 125
   Pro Gln Gly Ser Thr Asp Tyr Gly Ala Pro Thr Ser Phe Gly Ser Ser
       130                 135                 140
   Tyr Leu Pro Thr Pro Glu Pro Cys Glu Ala Trp Pro Pro Val Val Pro
   145                 150                 155                 160
   Val Ser Ser Val Thr Val Ser Ser Val Val Ser Ser Pro Ser Ser Ser
                   165                 170                 175
   Thr Gly His Pro Asp Glu Tyr Ala Ala Ser His Val Pro Thr Ser Val
               180                 185                 190
   Pro Ser Ser Leu Met Asp Ser Ser Val Met Gly Gln Ala Thr Gly Ile
           195                 200                 205
   Ser Cys Leu Asp Gly Met Lys Val Asn Tyr Asp Glu Ile Glu Ala Asp
       210                 215                 220
   Arg Gly Tyr Cys Pro Thr Ser Val Pro Gln Pro Gln Ser Ser Tyr Leu
   225                 230                 235                 240
   Pro Gln Gln Ser Trp Ser Met Tyr Pro Thr Ser Thr Tyr Tyr Pro Gln
                   245                 250                 255
   Ser Pro Thr Val
               260

## Description

La présente invention concerne un nouveau gène 763 indispensable à la pathogénie du champignon. L'invention a pour objet des polynucléotides 763, des polypetides 763, des organismes hôtes **non humains** exprimant un polypeptide 763 et leurs utilisations pour l'identification de nouvelles molécules antifongiques.

Le principe d'employer des gènes de champignons pathogènes, entièrement ou en partie, dans des tests pour identifier de nouvelles molécules actives contre ces champignons est connu en soi (in Antifungal Agents: Discovery and Mode of Action. Dixon GK, Coppong LG and Hollomon DW eds, BIOS Scientific publisher Ldt, Oxford, UK). Dans ce but, la connaissance du génome d'un champignon pathogène donné constitue une étape importante pour la réalisation de tels tests. Toutefois, la simple connaissance d'un gène donné n'est pas suffisante pour atteindre cet objectif, encore faut-il que le gène choisi comme cible de molécules fongicides potentielles soit essentiel à la vie du champignon, son inhibition par la molécule fongicide entraînant la mort du champignon ou essentiel à la pathogénie du champignon, son inhibition n'étant pas létale pour le champignon mais inhibant simplement son pouvoir pathogène. Cette deuxième catégorie de gènes cibles potentielles de molécules fongicides est particulièrement importante pour le développement d'une nouvelle génération de produits fongicides plus respectueux de l'environnement, s'attaquant de manière spécifique au seul pouvoir pathogène des champignons pathogènes.

La présente invention concerne l'identification et le clonage d'un nouveau gène 763 indispensable à la pathogénie du champignon. Un mutant 763 de *Magnaporthe grisea,* dans lequel le gène est inactivé, a une pathogénie réduite à 95%. Le gène 763 code pour un facteur de transcription comportant un motif de type bZIP, composé d'un motif à dominante basique de liaison séquence-spécifique à l'ADN suivi d'un autre dit "à fermeture-éclair à leucines", nécessaire à la dimérisation de la protéine. Ce motif est caractéristique d'une vaste famille de protéines régulatrices de l'expression des gènes. L'expression du gène 763 est détectée au cours des étapes précoces de l'infection de la plante. L'invention concerne également l'utilisation du gène 763 pour identifier de nouvelles molécules antifongiques et l'utilisation du gène 763 pour identifier d'autres gènes impliqués dans la pathogénie du champignon dont l'expression est régulée par 763.

### Description de la liste de séquences

SEQ ID No. 1: Gène 763 de *Magnaporthe grisea*
SEQ ID No. 2: ADNc du gène 763 de *Magnaporthe grisea*
SEQ ID No. 3: Polypeptide 763 de *Magnaporthe grisea*
SEQ ID No. 4: ADNc du gène 763 de *Neurospora crassa*
SEQ ID No. 5: Polypeptide 763 de *Neurospora crassa*

### Description de l'invention

### Polynucléotides

La présente invention concerne des polynucleotides comprenant un gène 763 de champignon. Le gène 763 peut être isolé chez les champignons phytopathogènes comme par exemple *Botrytis cinerea, Mycosphaerella graminicola, Stagnospora nodorum, Blumeria graminis, Colleotrichum lindemuthianum, Puccinia graminis, Leptosphaeria maculans, Fusarium oxysporum, Fusarium graminearum* et *Venturia inaequalis.* De manière avantageuse, le gène 763 est isolé chez les champignons phytopathogènes du genre *Magnaporthe* (Yu et al., 1999, database EMBL EBI, ACC No. AQ397193 15 mars 1999 ; AQ449199 9 avril 1991). De préférence, les polynucléotides de l'invention comprennent un gène 763 de *Magnaporthe grisea.* Préférentiellement, les polynucléotides de la présente invention comprennent la séquence codante d'un gène 763 de *Magnaporthe grisea.*

Le terme "polynucléotides 763" désigne l'ensemble des polynucléotides de la présente invention, de préférence, les polynucléotides de la séquence génomique de 763, les polynucléotides de la séquence de l'ADNc de 763, ainsi que les polynucléotides codant pour les polypeptides 763 de la présente invention. Le terme "polynucléotides 763" désigne également des polynucléotides recombinants comprenant les dits polynucléotides.

Selon la présente invention, on entend par "polynucléotide " une chaine nucléotidique simple brin ou son complémentaire ou une chaine nucléotidique double brin pouvant être de type ADN ou ARN. De préférence, les polynucléotides de l'invention sont de type ADN, notamment d'ADN double brin. Le terme "polynucléotide" désigne également les oligonucléotides et les polynucléotides modifiés.

Les polynucléotides de la présente invention sont isolés ou purifiés de leur environnement naturel. De préférence, les polynucléotides de la présente invention peuvent être préparés par les techniques classiques de biologie moléculaire telles que décrites par Sambrook et al. (Molecular Cloning : A Labratory Manual, 1989) ou par synthèse chimique.

L'invention concerne des polynucléotides comprenant la séquence génomique du gène 763 de *Magnaporthe grisea* de la SEQ ID No.1. Cette séquence génomique comprend 4 exons (positions 723-770, 925-1194, 1273-1554, 1663-1778 de la SEQ ID No. 1), 3 introns (positions 771-924, 1195-1272, 1555-1662 de la SEQ ID NO.1) et des séquences régulatrices en 5' et en 3'.

Dans un mode de réalisation préféré de l'invention, les polynucléotides de la séquence génomique de 763 comprennent un polynucléotide choisi parmi les polynucléotides suivants:
a) le polynucléotide de la SEQ ID No.1,
b) un polynucléotide comprenant au moins un exon de la SEQ ID No.1;
c) un polynucléotide comprenant une combinaison d'exons de la SEQ ID No.1.

La présente invention concerne également un polynucléotide comprenant une séquence régulatrice en 5' ou en 3' du gène 763 de *Magnaporthe grisea.* Dans un premier mode de réalisation, l'invention concerne un polynucléotide comprennent le promoteur du gène 763 de *Magnaporthe grisea* dont la séquence est comprise entre la position 1 et la position 705 de la SEQ ID No. 1. Dans un autre mode de réalisation, l'invention concerne un polynucléotide comprenant un fragment biologiquement actif du promoteur du gène 763 de *Magnaporthe grisea* dont la séquence est comprise entre la position 1 et la position 705 de la SEQ ID No. 1.

Par "fragment biologiquement actif' on entend ci-dessus un polynucléotide ayant une activité promotrice et plus particulièrement une activité promotrice dans les champignons. Les techniques permettant d'évaluer l'activité promotrice d'un polynucléotide sont bien connues de l'homme du métier. Ces techniques impliquent classiquement l'utilisation d'un vecteur d'expression comprenant dans le sens de la transcription le polynucléotide à tester et un gène rapporteur (voir Sambrook et al., Molecular Cloning : A Labratory Manual, 1989).

L'invention concerne aussi des polynucléotides comprenant l'ADNc de 763 de *Magnaporthe grisea* de la SEQ ID No. 2. L'ADNc du gène 763 *de Magnaporthe grisea* comporte la séquence codante du gène 763 ainsi qu' une séquence régulatrice 5' UTR et une séquence régulatrice 3' UTR. L'invention concerne plus particulièrement des polynucléotides comprenant la séquence codante du gène 763 de *Magnaporthe grisea* dont la séquence est comprise entre la position 17 et la position 733 de la SEQ ID No. 2.

L'invention s'étend également aux polynucléotides comprenant un polynucléotide choisi parmi les polynucléotides suivants:
a) le polynucléotide de la SEQ ID No. 1;
b) le polynucléotide de la SEQ ID No. 2;
c) le polynucléotide de la SEQ ID No. 4;
d) un polynucléotide homologue à un polynucléotide tel que défini en a) ou b) ou c);
e) un polynucléotide capable de s'hybrider de manière sélective à un polynucléotide tel que défini en a) ou b) ou c).

Par " homologue " on entend selon l'invention un polynucléotide présentant une ou plusieurs modifications de séquence par rapport à la séquence de référence. Ces modifications peuvent être des délétions, des additions ou des substitutions d'un ou plusieurs nucléotides de la séquence de référence. De manière avantageuse, le pourcentage d'homologie sera d'au moins 70 %, 75%, 80%, 85%, 90%, 95% et de préférence d'au moins 98% et plus préférentiellement d'au moins 99% par rapport à la séquence de référence. Les méthodes de mesure et d'identification des homologies entre les séquences d'acides nucléiques sont bien connues de l'homme du métier. On peut employer par exemple les programmes PILEUP ou BLAST (notamment Altschul et al., J.Mol.Evol. 36:290-300, 1993; Altschul et al., J.Mol.Biol. 215 :403-10, 1990; Altschul et al., NAR 25:3389-3402, 1997). De préférence, on utilisera les paramètres par défaut. L'invention concerne donc des polynucléotides comprenant des polynucléotides présentant au moins 70 %, 75%, 80%, 85%, 90%, 95%, 98% et de préférence au moins 98% et plus préférentiellement au moins 99% d'homologie avec les polynucléotides 763, les polynucléotides des SEQ ID No. 1-2 ou le polynucléotides de la SEQ ID No. 4. De préférence, l'invention concerne un polynucléotide comprenant un polynucléotide d'au moins 50, 100, 200, 300, 400, 500, 1000 nucléotides présentant au moins 70 %, 75%, 80%, 85%, 90%, 95%, 98% et de préférence au moins 98% et plus préférentiellement au moins 99% d'homologie avec les polynucléotides 763, les polynucléotides des SEQ ID No.1-2 ou les polynucléotides de la SEQ ID No. 4. De préférence, les polynucléotides homologues à un polynucléotide de référence conservent la fonction de la séquence de référence.

Par "séquence capable de s'hybrider de manière sélective ", on entend selon l'invention les séquences qui s'hybrident avec la séquence de référence à un niveau supérieur au bruit de fond de manière significative. Le niveau du signal généré par l'interaction entre la séquence capable de s'hybrider de manière sélective et les séquences de référence est généralement 10 fois, de préférence 100 fois plus intense que celui de l'interaction des autres séquences d'ADN générant le bruit de fond. Les conditions d'hybridation stringentes permettant une hybridation sélective sont bien connues de l'homme du métier. En général la température d'hybridation et de lavage est inférieure d'au moins 5°C au Tm de la séquence de référence à un pH donné et pour une force ionique donnée. Typiquement la température d'hybridation est d'au moins 30°C pour un polynucléotide de 15 à 50 nucléotides et d'au moins 60°C pour un polynucléotide de plus de 50 nucléotides. A titre d'exemple, l'hybridation est réalisée dans le tampon suivant: 6X SSC, 50 mM Tris-HCl (pH 7.5), 1 mM EDTA, 0.02% PVP, 0.02% Ficoll, 0.02% BSA, 500 µg/ml denatured salmon sperm DNA. Les lavages sont par exemple réalisés successivement à faible stringence dans un tampon 2X SSC, 0,1%SDS, à moyenne stringence dans un tampon 0,5X SSC, 01%SDS et à forte stringence dans un tampon 0,1X SSC, 0,1%SDS. L'hybridation peut bien entendu être effectuée selon d'autres méthodes usuelles bien connues de l'homme du métier (voir notamment Sambrook et al., Molecular Cloning : A Labratory Manual, 1989). L'invention concerne donc des polynucléotides comprenant un polynucléotide capable de s'hybrider de manière sélective avec le polynucléotide des SEQ ID Nos. 1-2 ou le polynucléotide de la SEQ ID No. 4. De préférence, l'invention concerne un polynucléotide comprenant un polynucléotide d'au moins 50, 100, 200, 300, 400, 500, 1000 nucléotides capable de s'hybrider de manière sélective avec le polynucléotide de la SEQ ID Nos.1-2 ou le polynucléotide de la SEQ ID No. 4. De préférence, les polynucléotides s'hybridant de manière sélective à un polynucléotide de référence conservent la fonction de la séquence de référence.

De préférence, les polynucléotides de la présente invention conservent la fonction du gène 763 de *Magnaporthe grisea* et codent pour un facteur de transcription essentiel à la pathogénie du champignon exprimé au début du stade infectieux.

Préférentiellement, les polynucléotides de la présente invention complémentent un mutant 763 de *Magnaporthe grisea* et restaurent sa pathogenicité pour le riz et l'orge. Un mutant 763 selon l'invention est un mutant de *Magnaporthe grisea* dans lequel le gène 763 de la SEQ ID No. 1 est inactivé par des techniques bien connues de l'homme du métier.

La présente invention concerne également des variants allèliques ou des homologues du gène 763 de *Magnaporthe grisea.*

La présente invention concerne également l'identification et le clonage de gènes homologues au gène 763 de *Magnaporthe grisea* chez d'autres champignons phytopathogènes. De préférence, ces gènes homologues sont susceptibles d'être isolés ou clonés à partir d'un champignon phytopathogène choisi parmi *Botrytis cinerea, Mycosphaerella graminicola, Stagnospora nodorum, Blumeria graminis, Colleotrichum lindemuthianum, Puccinia graminis, Leptosphaeria maculans, Fusarium oxysporum, Fusarium graminearum* et *Venturia inaequalis.* L'invention a ainsi pour objet l'utilisation d'un polynucléotide ou d'un fragment d'un polynucléotide de la SEQ ID No. 1 et de la SEQ ID No. 2 selon l'invention pour l'identification de gènes homologues dans d'autres champignons phytopathogènes. Les techniques permettant le clonage de gènes 763 homologues chez d'autres champignons phytopathogènes sont bien connues de l'homme du métier. Le clonage s'effectue par exemple par criblage de banques d'ADNc ou de banques d'ADN génomique avec un polynucléotide ou un fragment d'un polynucléotide de la SEQ ID No.1 et de la SEQ ID No.2. Ces banques peuvent également être criblés par PCR à l'aide d'oligonucléotides spécifiques ou dégénérés dérivés de la SEQ ID No.1 ou de la SEQ ID No.2. Les techniques de construction et de criblage de ces banques sont bien connues de l'homme du métier (voir notamment Sambrook et al., Molecular Cloning : A Labratory Manual, 1989). Des gènes 763 de champignon phytopathogène peuvent également être identifiés dans les bases de données par BLAST nucléotidique ou protéique à l'aide des SEQ ID NO. 1-3.

De préférence, les gènes clonés conservent la fonction du gène 763 de *Magnaporthe grisea* et codent pour un facteur de transcription essentiel à la pathogénie du champignon exprimé au début du stade infectieux. Les séquences des gènes clonées peuvent être analysées selon des méthodes connues afin d'établir qu'ils codent pour un facteur de transcription de champignon et notamment afin d'établir qu'ils codent pour un polypeptide comprenant un motif de type bZIP, composé d'un motif à dominante basique de liaison séquence-spécifique à l'ADN suivi d'un autre dit "à fermeture-éclair à leucines", nécessaire à la dimérisation de la protéine. Par ailleurs, les techniques permettant d'établir qu'un gène connu est essentiel à la pathogénie d'un champignon sont connues de l'homme du métier. Par exemple, le gène étudié est inactivé dans le champignon par des techniques classiques de biologie moléculaire, on citera notamment le remplacement du gène par un gène marqueur par recombinaison homlogue. La réduction de la pathogénie du champignon comportant le gène inactivé est analysée par des tests phénotypiques. De préférence, l'inactivation du gène homologue provoque une réduction de la pathogénie d'au moins 95%. Les techniques pour analyser l'expression d'un gène dans les différents stades de développement du champignon et plus particulièrement au début de l'infection sont également bien connues de l'homme du métier. Typiquement, on prépare des ARN totaux ou des ARNm (poly A+) à partir des différents stades de développement du champignon. Ces ARN sont ensuite analysés par RT-PCR ou par Northern Blot afin de déterminer le niveau d'expression du gène. D'autres techniques bien connues de l'homme du métier peuvent être utilisées afin d'établir que les polynucléotides de l'invention conservent la fonction du gène 763 de *Magnaporthe grisea.* On citera notamment la complémentation de mutants 763 suivis de tests de restauration de la pathogénie du champignon.

Une recherche dans les bases de données par blast a permis d'identifier un homologue du gène 763 de *Magnaporthe grisae* chez *Neurospora crassa.* Ce nouveau gène a été identifié par blast de séquences génomiques non annotées. L'ADNc de ce gène 763 de Neurospora correspond à la SEQ ID No. 4 et le polypeptide 763 de *Neurospora* à la SEQ ID No. 5.

L'invention a également pour objet des polynucléotides comprenant un polynucléotide codant pour un polypeptide choisi parmi les polypeptides suivants:
a) le polypeptide de la SEQ ID No. 3;
b) le polypeptide de la SEQ ID No. 5;
c) un polypeptide homologue à un polypeptide tel que défini en a) ou b);
c) un fragment biologiquement actif d'un polypeptide tel que défini en a) ou b).

### Polypeptides

La présente invention concerne également des polypeptides 763 de champignon phytopathogène et plus particulièrement de *Magnaporthe grisea.* Le terme "polypeptides 763" désigne l'ensemble des polypeptides de la présente invention ainsi que les polypeptides pour lesquels codent les polynucléotides de la présente invention. Le terme "polypeptides 763" désigne également des protéines de fusion, des protéines recombinantes ou des protéines chimères comprenant ces polypeptides. Dans la présente description le terme "polypeptide" désigne également des protéines et des peptides ainsi que des polypeptides modifiés.

Les polypeptides de l'invention sont isolés ou purifiés de leur environnement naturel. Les polypeptides peuvent être préparés au moyen de différents procédés. Ces procédés sont notamment la purification à partir de sources naturelles telles que des cellules exprimant naturellement ces polypeptides, la production de polypeptides recombinants par des cellules hôtes non humaines appropriées et leur purification ultérieure, la production par synthèse chimique ou, enfin, une combinaison de ces différentes approches. Ces différents procédés de production sont bien connus de l'homme du métier. Ainsi, les polypeptides 763 de la présente invention peuvent être isolés à partir de champignons exprimant des polypeptides 763. De préférence, les polypeptides 763 de la présente invention sont isolés à partir d'organismes hôtes non humains recombinants exprimant un polypeptide 763 hétérologue. Ces organismes sont préférentiellement choisis parmi les bactéries, les levures, les champignons, les cellules animales ou d'insectes.

La présente invention a pour objet un polypeptide comprenant un polypeptide 763 de *Magnaporthe grisea* de la SEQ ID No. 3. L'invention concerne également des polypeptides comprenant un fragment biologiquement actif ou un homologue du polypeptide 763 de la SEQ ID No. 3.

Dans un autre mode de réalisation, la présente invention a pour objet un polypeptide comprenant un polypeptide 763 de *Neurospora crassa* de la SEQ ID No. 5. L'invention concerne également des polypeptides comprenant un fragment biologiquement actif ou un homologue du polypeptide 763 de la SEQ ID No. 5.

Le terme "fragment" d'un polypeptide désigne un polypeptide comprenant une partie mais pas la totalité du polypeptide dont il est dérivé. L'invention concerne un polypeptide comprenant un fragment d'au moins 10, 15, 20, 25, 30, 35, 40, 50, 100, 200 acides aminés d'un polypeptide de la SEQ ID NO.3.

Le terme "fragment biologiquement actif" désigne un fragment d'un polypeptide conservant la fontion du polypeptide dont il est dérivé. Les fragments biologiquement actifs du polypeptide de la SEQ ID No. 3 conservent ainsi la fonction du polypeptide 763 de *Magnaporthe grisea.* Ces fragments biologiquement actifs ont donc une activité de facteur de transcription fonctionnel dans les champignons. Préférentiellement, cette activité est essentielle à la pathogénie du champignon.

Le terme "homologue" désigne un polypeptide pouvant présenter une délétion, une addition ou une substitution d'au moins un acide aminé. L'invention a pour objet un polypeptide présentant au moins 75%, 80%, 85%, 90%, 95%, 98% et préférentiellement au moins 99% d'acides aminés identiques avec un polypeptide de la SEQ ID No. 3 ou de la SEQ ID No. 5. Les méthodes de mesure et d'identification des homologies entre polypeptides ou protéines sont connues de l'homme du métier. On peut employer par exemple le « package » UWGCG et le programme BESTFITT pour calculer les homologies (Devereux et al., Nucleic Acid Res. 12, 387-395, 1984). De préférence, on utilise les paramètres par défaut.

De préférence, ces polypeptides homologues conservent la même activité biologique que le polypeptide 763 de *Magnaporthe grisea* de la SEQ ID No.3. Préférentiellement, ces polypeptides ont donc une activité de facteur de transcription de champignon. Préférentiellement, cette activité est essentielle à la pathogénie du champignon. Dans un mode de réalisation préféré, ces polypeptides homologues sont suceptibles d'être isolés à partir de champignons phytopathogènes. De préférence, ces polypeptides sont exprimés dans les champignons phytopathogènes au début de l'infection de la plante.

L'invention a également pour objet un polypeptide de fusion comprenant un polypeptide 763 tel que décrit ci-dessus fusionné à un polypeptide rapporteur. Le polypeptide rapporteur permet la détection rapide de l'expression d'un polypeptide 763 dans un champignon ou dans un autre organisme hôte **non humain.** Parmi les polypeptides pouvant ainsi être fusionés avec un polypeptide 763 on citera notamment la GFP (green fluorescent protein) et la protéine GUS (β-glucuronidase). Ces protéines de fusion et leurs constructions sont bien connues de l'homme du métier.

### Cassettes d'expression, vecteurs et organismes hôtes non humains

Le gène 763 peut être exprimé dans différents organismes hôtes non humains tels que les bacteries, les levures, les champignons, les cellules animales ou d'insectes. Le gène 763 peut être exprimé dans un organisme hôte non humain sous le contrôle du promoteur 763 de la présente invention ou sous le contrôle d'un promoteur hétérologue.

### Cassettes d'expression

Selon un mode de réalisation de l'invention, un polynucléotide codant pour un polypeptide 763 est inséré dans une cassette d'expression en utilisant des techniques de clonage bien connues de l'homme du métier. Cette cassette d'expression comprend les éléments nécessaires à la transcription et à la traduction des séquences codant pour le polypeptide 763. Avantageusement, cette cassette d'expression comprend à la fois des éléments permettant de faire produire un polypeptide 763 par une cellule hôte **non humain** et des éléments nécessaires à la régulation de cette expression. Dans un premier mode de réalisation, les cassettes d'expression selon l'invention comprennent, dans le sens de la transcription, un promoteur fonctionnel dans un organisme hôte **non humain**, le gène 763 ou la séquence codante du gène 763 et une séquence terminatrice dans ledit organisme **non humain**. Préférentiellement, la cassette d'expression comprend, dans le sens de la transcription, un promoteur fonctionnel dans un organisme hôte **non humain**, un polynucléotide choisi parmi les polynucléotides suivants:
a) un polynucléotides codant pour le polypeptide 763 de la SEQ ID No. 3 ou pour un fragment biologiquement actif du polypeptide 763 de la SEQ ID No.3;
b) un polynucléotide dont la séquence est comprise entre la position 17 et la position 733 de la SEQ ID No.2;
c) un polynucléotide de la SEQ ID No. 1;
d) un polynucléotide de la SEQ ID No. 2;
e) un polynucléotides codant pour le polypeptide 763 de la SEQ ID No. 5 ou pour un fragment biologiquement actif du polypeptide 763 de la SEQ ID No.5;
f) un polynucléotide de la SEQ ID No. 4;
g) un polynucléotide homologue à un polynucléotide tel que défini en b), c), d) ou f);
h) un polynucléotide capable de s'hybrider de manière spécifique à un polynucléotide tel que défini en b), c), d) ou f);
et une séquence terminatrice dans ledit organisme hôte **non humain.**

Tout type de séquence promotrice peut être utilisée dans les cassettes d'expression selon l'invention. Le choix du promoteur dépendra notamment de l'organisme hôte **non humain** choisi pour l'expression du gène d'intérêt. Certains promoteurs permettent une expression constitutive alors que d'autres promoteurs sont au contraire inductibles. Parmi les promoteurs fonctionnels dans les champignons, on citera notamment celui de glyceraldehyde-3-phosphate deshydrogenase d'*Aspergillus nidulans* (Roberts et al., Current Genet. 15:177-180, 1989). Parmi les promoteurs fonctionnels dans les bactéries, on citera notamment celui de la RNA polymérase du bacteriophage T7 (Studier et al., Methods in enzymology 185:60-89, 1990). Parmi les promoteurs fonctionnels dans les levures, on citera notamment celui du gène Gal1 (Elledge et al., Proc Natl Acad Sciences, USA. 88:1731-1735, 1991) ou les promoteurs GAL4 et ADH de *S.cerevisiae.* Parmi les promoteurs fonctionnels dans les cellules d'insectes, on citera notamment le promoteur de la polyhédrine de baculovirus AcMNPV (Weyer et al., J.Gene.Virol. 72:2967-2974, 1991). Parmi les promoteurs fonctionnels dans les cellules animales on citera le promoteur de la metallothionein et les promoteurs de virus et d'adénovirus. Tous ces promoteurs sont décrits dans la littérature et bien connus de l'homme du métier.

Le promoteur 763 peut être utilisé pour exprimer un gène hétérologue dans un organisme hôte non humain et notamment dans les champignons. L'invention a donc également pour objet des cassettes d'expression comprenant le promoteur d'un gène 763 associé de manière fonctionnelle à une séquence codant pour une protéine hétérologue, permettant l'expression de ladite protéine dans les champignons. De préférence, la cassette d'expression selon l'invention comprend, dans le sens de la transcription, un polynucléotide dont la séquence est comprise entre la position 1 et la position 705 de la SEQ ID No. 1 ou un fragment biologiquement actif du polynucléotide dont la séquence est comprise entre la position 1 et la position 705 de la SEQ ID NO. 1, la séquence codant pour un polypeptide hétérologue et une séquence terminatrice fonctionnelle dans champignons. Tout gène d'intérêt peut être exprimé dans un organisme hôte **non humain** sous le contrôle d'un promoteur 763. De préférence, le promoteur 763 est utilisé pour l'expression d'un gène hétérologue dans les champignons. L'activité du promoteur 763 dans différentes conditions peut être évalué à l'aide d'un gène rapporteur tel que le gène rapporteur GUS (β-glucuronidase), GFP (green fluorescent protein), LUC (luciferase), CAT (chloramphenicol transferase) ou β-galactosidase (lacZ).

Dans un mode de réalisation préféré de l'invention, le promoteur 763 est associé de manière fonctionnelle à la séquence codante d'un gène marqueur. L'expression du gène marqueur permet la sélection des organismes transformés par leur résistance aux antibiotiques ou aux herbicides par exemple. On citera notamment les séquences codantes pour un gène de tolérance à un antibiotique ou un herbicide, comme les gènes de résistance à l'hygromycine (hph : Punt et al., 1987), à la phléomycine (ble : Drocourt, 1990) ou à l'herbicide bialaphos (Bar : Pall et Brunelli, 1993).

Les cassettes d'expression, selon la présente invention, peuvent en outre inclure toute autre séquence nécessaire à l'expression du gène 763 ou du gène hétérologue, comme par exemple des éléments de régulation ou des séquences signal permettant l'adressage du polypeptide 763. On peut notamment utiliser toute séquence de régulation permettant d'augmenter le niveau d'expression de la séquence codante insérée dans ladite cassette d'expression. Selon l'invention, on peut notamment utiliser, en association avec la séquence de régulation promotrice, d'autres séquences de régulation, qui sont situées entre le promoteur et la séquence codante, telles que des activateurs de transcription ("enhancer"). Comme signal d'adressage membranaire dans les organismes hôtes **non humains** on citera notamment celui de la proteine A chez les bactéries (Nilsson et al., Methods in Enzymology 198:3, 1991).

Une grande variété de séquences terminatrices sont utilisables dans les cassettes d'expression selon l'invention, ces séquences permettent la terminaison de la transcription et la polyadénylation de l'ARNm. Toute séquence terminatrice fonctionnelle dans l'organisme hôte **non humain** sélectionné peut être utilisée.

La présente invention a également pour objet un polynucléotide comprenant une cassette d'expression selon l'invention, avantageusement les cassettes d'expression selon la présente invention sont insérées dans un vecteur.

### Vecteurs

La présente invention concerne donc également des vecteurs de réplication ou d'expression pour la transformation d'un organisme **non humain** comprenant au moins un polynucléotide 763 ou une cassette d'expression selon la présente invention. Ce vecteur peut notamment être constitué par un plasmide, un cosmide, un bactériophage ou un virus dans lequel est inséré un polynucléotide 763 ou une cassette d'expression selon l'invention. Les techniques de construction de ces vecteurs et d'insertion d'un polynucléotide de l'invention dans ces vecteurs sont bien connues de l'homme du métier. De manière générale, tout vecteur capable de se maintenir, de s'autorépliquer ou de se propager dans une cellule hôte non humaine et notamment afin d'induire l'expression d'un polynucléotide ou d'un polypeptide peut être utilisé. Avantageusement, les vecteurs selon l'invention comprennent au moins une origine de réplication pour leur réplication dans un organisme hôte **non humaine**. De manière préférée, les vecteurs de l'invention comprennent également au moins un marqueur de sélection tel qu'un gène de résistance à un antibiotique. On citera notamment des vecteurs tels que pBluescript (Stratagene, La Jolla, Ca), pTrcHis (Invitrogen, La Jolla, Ca) et des des vecteurs d'expression derivés de baculovirus tels que ceux dérivés du virus de la polyhédrine d'*Autographica californica* (AcMNPV). Un système préfére combinant un baculovirus et une cellule d'insecte est le système baculovirus pV111392/ cellules Sf21 (Invitrogen, la Jolla, Ca). Pour l'expression dans les cellules animales on utilise notamment des vecteurs dérivés d'adénovirus. L'homme du métier choisira les vecteurs appropriés notamment en fonction de l'organisme hôte **non humain** à transformer et en fonction de la technique de transformation mise en oeuvre. Les méthodes de transformation des organismes hôtes **non humains** sont bien connus de l'homme du métier (Inoue et al., Gene 96:23-28, 1990; Fincham, Microbiological Reviews 53:148-170, 1989)

Les vecteurs de la présente invention sont notamment utilisés pour transformer un organisme **non humain** en vue de la réplication du vecteur et/ou de l'expression d'un polypeptide 763 dans ledit organisme hôte **non humain**. L'invention concerne une méthode pour préparer un polypeptide M763 comprenant les étapes suivantes:
- on transforme un organisme hôte **non humain** avec un vecteur d'expression comprenant une cassette d'expression selon l'invention,
- on isole les polypeptides M763 produits par l'organisme hôte non humain.

Les polypeptides 763 recombinants produits par un organisme hôte **non humain** transformé avec un polynucléotide peuvent être purifiés ou isolés selon des méthodes connues de l'homme du métier. Les polypeptides M763 peuvent être exprimés dans un organisme hôte **non humain** sous la forme de protéines de fusion. On citera notamment les vecteurs pGEX pour l'expression de protéines de fusion comprenant la glutathione S-transférase (GST). Ces protéines de fusion sont facilement purifiées par adsorption sur des billes de glutathione-agarose. Le groupement GST peut ensuite être éliminé par digestion avec la protéase Xa. D'autres systèmes pour l'expression et la purification de protéines de fusion sont connues de l'homme du métier.

### Organismes hôtes non humains

La présente invention a également pour objet, un procédé de transformation d'un organisme hôte **non humain** par intégration dans ledit organisme hôte **non humain** d'au moins un polynucléotide 763 ou d'une cassette d'expression ou d'un vecteur selon l'invention. Le polynucléotide peut être intégré dans le génome de l'organisme hôte **non humain** ou se repliquer de manière stable dans l'organisme hôte non humain. Les méthodes de transformation des organismes hôtes **non humains** sont bien connus de l'homme du métier et largement décrits dans la littérature (Inoue et al., Gene 96:23-28, 1990 ; Fincham, Microbiological Reviews 53:148-170,1989).

La présente invention concerne également un organisme hôte **non humain** transformé avec un polynucléotide 763, une cassette d'expression ou un vecteur selon l'invention. Par organisme hôte **non humain**, on entend en particulier selon l'invention tout organisme mono ou pluricellulaire, inférieur ou supérieur, en particulier choisi parmi les bactéries, les levures, les champignons, les cellules animales et les cellules d'insectes. De manière avantageuse, les bactéries sont choisies parmi *Escherichia coli* et *Bacillus subtilis,* les levures sont choisies parmi *Pichia pastoris* et *Saccharomyces cerevisae,* les cellules d'insectes sont choisis parmi *Spodoptera frugiperda* et *Drosophila melanogaster,* les cellules animales sont choisis parmi les cellules CHO, HeLa et COS.

Les techniques de construction de vecteurs, de transformation d'organismes hôtes **non humains** et d'expression de protéines hétérologues dans ces organismes sont largement décrites dans la littérature (Ausubel F.M. et al., "Current Protocols in Molecular Biology" Volumes 1 et 2, Greene Publishing Associates et Wiley -Interscience, 1989; T.Maniatis, E.F.Fritsch, J.Sambrook, Molecular Cloning A laboratory Handbook, 1982).

La présente invention concerne également l'utilisation de polynucléotides 763 et de polypeptides 763 pour l'identification des gènes impliqués dans la pathogénie des champignons ou pour l'identification de nouvelles molécules fongicides inhibitrices de la pathogénie des champignons.

### Inhibition de la pathogénie des champignons

Des champignons dans lesquels le gène 763 est inactivé ou inhibé ont une pathogénie réduite de 95%. L'invention concerne des procédés pour inhiber la pathogénie des champignons en inactivant ou en inhibant l'expression du gène 763. De préférence, les champignons sont choisis parmi *Botrytis cinerea, Mycosphaerella graminicola, Stagnospora nodorum, Blumeria graminis, Colleotrichum lindemuthianum, Puccinia graminis, Leptosphaeria maculans, Fusarium oxysporum, Fusarium graminearum* et *Venturia inaequalis.*

De manière préférée, l'invention concerne des procédés pour inhiber la pathogénie d'un champignon, les dits procédés comprenant l'inhibition de l'expression d'un polynucléotide 763 selon l'invention dans ledit champignon, ou l'inhibition de l'expression d'un polypeptide 1763 selon l'invention dans ledit champignon ou l'inhibition de l'activité biologique d'un polypeptide 763 selon l'invention dans ledit champignon. De préférence, cette inhibition affecte spécifiquement l'expression du gène 763 et l'activité biologique du polypeptide 763. L'invention ne concerne donc pas les procédés comprenant l'inhibition générale de l'expression des gènes dans le champignon. On comprendra que l'inhibition de l'expression du gène 763 peut cependant entrainer l'inhibition d'autres gènes.

Différentes méthodes bien connues de l'homme du métier peuvent être mises en oeuvre pour inhiber la pathogénie des champignons en inhibant l'expression du gène 763 dans ces champignons. Dans un mode de réalisation de l'invention, le gène 763 est inactivé par mutagenèse insertionnelle ou par recombinaison homologue (techniques de remplacement de gène ou de "knock out"). Dans un autre mode de réalisation de l'invention, l'expression d'un polypeptide 763 est inhibée par l'expression d'un polynucléotide antisens du gène 763 dans les champignons. Dans un troisième mode de réalisation de l'invention, l'expression du gène 763 est inhibée par un composé inhibiteur.

Le niveau d'expression d'un polynucléotide 763 ou d'un polypeptide 763 dans les champignons peut être mesuré selon des techniques décrites dans la littérature. On citera notamment le Northern blot, la PCR et les DNA arrays (puces à ADN) pour les polynucléotides et le Western blot pour les polypeptides. Ces techniques sont bienb connues de l'homme du métier.

### Identification de nouvelles molécules fongicides inhibitrices de la pathogénie des champignons

L'inactivation du gène 763 chez *Magnaporthe grisea,* champignon pathogène du riz, entraine une réduction de 95% de la pathogénie de ce champignon. Par ailleurs, des gènes homologues ont pu être identifiés chez d'autres champignons. Par conséquent, des composés inhibiteurs de l'expression du gène 763 ou de l'activité du polypeptide 763 dans les champignons peuvent être utilisés pour inhiber la pathogénie des champignons.

L'invention concerne donc des procédés pour l'identification de composés inhibant la pathogénie des champignons comprenant une étape d'identification d'un composé inhibant spécifiquement l'expression d'un polynucléotide 763 dans ledit champignon, ou une étape d'identification d'un composé inhibant l'expression d'un polypeptide 763 dans ledit champignon ou une étape d'identification d'un composé inhibant l'activité biologique d'un polypeptide 763 dans ledit champignon.

De préférence, les champignons sont choisis parmi *Botrytis cinerea, Mycosphaerella graminicola, Stagnospora nodorum, Blumeria graminis, Colleotrichum lindemuthianum, Puccinia graminis, Leptosphaeria maculans, Fusarium oxysporum, Fusarium graminearum et Venturia inaequalis.*

Les polynucléotides 763, les polypeptides 763, les vecteurs et les organismes hôtes **non humains** de la présente invention peuvent ainsi être utilisés dans différents test de criblage afin d'identifier de nouveaux composés antifongiques.

### Identification d'inhibiteurs se fixant sur la protéine 763

Des molécules inhibant directement l'activité du polypeptide 763 pourraient inhiber la pathogénie du champignon et conduire au développement de nouveaux fongicides.

L'invention concerne donc un procédé pour l'identification de composés inhibant la pathogénie des champignons comprenant les étapes suivantes:
- mettre en contact ledit composé avec un polypeptide 763, et
- détecter la fixation dudit composé audit polypeptide; et
Préférentiellement, le procédé comprend également une étape dans laquelle on détermine si ledit composé inhibe la pathogénie des champignons.

Toute méthode permettant de préparer un polypeptide 763 et de le purifier ou de l'isoler peut être utilisée dans les procédés de la présente invention.

De préférence, le polypeptide763 est exprimée dans un système d'expression hétérologue (par exemple bactérie, levure, cellule animale ou d'insecte) au moyen d'un polynucléotide 763 selon l'invention, la purification simplifiée du polypeptide 763 permet ensuite d'identifier de nouvelles molécules se fixant sur la protéine 763. L'identification des dites molécules se fait par des méthodes bien connues de l'homme du métier, notamment des méthodes de détection physique de la fixation des composés testés sur la protéine 763 (système BIACORE; Karlson & al., J. of Biomolecular Interaction Analysis, special issue Drug Discovery : 18-22).

### Identification d'inhibiteurs des régulateurs de l'expression du gène 763

Des molécules inhibant l'expression du gène 763 peuvent également inhiber la pathogénie du champignon et conduire au développement de nouveaux fongicides. Dans la présente invention, l'expression "inhibition de l'expression du gène 763" désigne l'inhibition de l'expression d'un polynucléotide 763 ainsi que l'inhibition de l'expression d'un polypeptide 763 dans les organismes hôtes **non humains** et préférentiellement dans les champignons phytopathogènes.

L'invention a également pour objet un procédé pour l'identification de composés inhibant la pathogénie des champignons comprenant les étapes suivantes:
- mettre en contact ledit composé avec un organisme hôte **non humain** transformé avec un polynucléotide ou un vecteur selon l'invention tel que cet organisme hôte **non humain** exprime un gène rapporteur sous le contrôle du promoteur du gène 763; et
- détecter l'inhibition de l'expression dudit gène rapporteur.
Préférentiellement, le procédé comprend également une étape dans laquelle on détermine si ledit composé inhibe la pathogénie des champignons.

L'utilisation d'un polynucléotide selon l'invention comprenant le promoteur 763 associé à la séquence codante d'un gène reporteur (GUS ou GFP par exemple) permet de mesurer l'activité promotrice du promoteur 763 dans une cellule fongique ou dans une cellule hôte **non humaine**. Ce procédé permet d'identifier des composés inhibant l'activité du promoteur 763 et donc l'expression du gène 763 au niveau transcriptionnel. Une souche recombinée comprenant le gène ci-dessus est ainsi utilisée pour identifier des molécules inhibant l'expression du gène 763, ce qui se manifeste par une inhibition de l'expression de la protéine rapporteur de la souche recombinée dans les conditions d'expression du gène 763. Ce type de test est bien connu de l'homme du métier et décrit dans la littérature, notamment Axiotis et al. (1995. pp. 1-7 in Antifungal Agents: Discovery and Mode of Action. Dixon GK, Coppong LG and Hollomon DW eds, BIOS Scientific publisher Ldt, Oxford, UK).

Dans un autre mode de réalisation, l'invention concerne un procédé pour l'identification de composés inhibant la pathogénie des champignons comprenant les étapes suivantes:
- mettre en contact ledit composé avec un organisme hôte **non humain** transformé avec un polynucléotide selon l'invention ou un vecteur selon l'invention, ledit organisme hôte **non humain** exprimant un polypeptide 763; et
- détecter l'inhibition de l'expression dudit polypeptide 763.

De préférence, le polypeptide 763 est un polypeptide de fusion comprenant un polypeptide rapporteur tel que GUS ou GFP dont l'expression est facilement mesurée. Préférentiellement, le procédé comprend également une étape dans laquelle on détermine si ledit composé inhibe la pathogénie des champignons. Ce procédé permet d'identifier des composés inhibant l'expression du gène 763 au niveau transcriptionnel ou au niveau traductionnel. Une souche recombinée exprimant un polypeptide 763 et de préférence un polypeptide 763 fusionné à un rapporteur est ainsi utilisée pour identifier des molécules inhibant l'expression du gène 763, ce qui se manifeste par une inhibition de l'expression du polypeptide 763 de la souche recombinée dans les conditions d'expression du gène 763.

La présente invention concerne donc un procédé pour identifier des composés inhibant la pathogénie des champignons liée à l'expression du gène 763, ledit procédé consistant à soumettre un composé, ou un mélange de composés, à un test approprié pour l'identification des composés inhibiteurs de ladite pathogénie des champignons et à sélectionner les composés réagissant de manière positive audit test, le cas échéant à les isoler, puis à les identifier.

De manière préférentielle, le test approprié est un test tel que défini ci-dessus.

De manière préférée, un composé identifie selon ces procédés est ensuite testé pour ces propriétés anti-fongiques et pour sa capacité à inhiber la pathogénie du champignon pour les plantes selon des méthodes connues de l'homme du métier. Préférentiellement, le composé est évalué à l'aide de tests phénotypiques tels que des essais de pathogénie sur feuilles ou sur plantes entières.

Par composé on entend selon l'invention tout composé chimique ou mélange de composés chimiques, y compris les peptides et les protéines.

Par mélange de composés on comprend selon l'invention au moins deux composés différents, comme par exemple les (dia)stéréoisomères d'une molécule, des mélanges d'origine naturelle issus de l'extraction de matériel biologique (plantes, tissus végétaux, culture bactériennes, cultures de levures ou de champignons, insectes, tissus animaux, etc.) ou des mélanges réactionnels non purifiés ou purifiés totalement ou en partie, ou encore des mélanges de produits issus de techniques de chimie combinatoire.

La présente invention concerne enfin de nouveaux composés inhibiteurs de la pathogénie des champignons liée à l'expression du gène 763, notamment les composés identifiées par le procédé selon l'invention et/ou les composés dérivés des composés identifiés par le procédé selon l'invention.

De manière préférentielle, les composés inhibiteurs de la pathogénie des champignons liée à l'expression du gène 763 ne sont pas des inhibiteurs généraux d'enzymes. De manière également préférentielle, les composés selon l'invention ne sont pas des composés déjà connus pour avoir une activité fongicide et/ou une activité sur la pathogénie des champignons.

Les exemples ci-après permettre d'illustrer l'invention, sans toutefois chercher à en limiter la portée.
Toutes les méthodes ou opérations décrites ci-dessous dans ces exemples sont données à titre d'exemples et correspondent à un choix, effectué parmi les différentes méthodes disponibles pour parvenir au même résultat. Ce choix n'a aucune incidence sur la qualité du résultat et par conséquent, toute méthode adaptée peut être utilisée par l'homme de l'art pour parvenir au même résultat. La plupart des méthodes d'ingénierie des fragments d'ADN sont décrites dans "Current Protocols in Molecular Biology" Volumes 1 et 2, Ausubel F.M. et al , publiés par Greene Publishing Associates et Wiley -Interscience (1989) ou dans Molecular cloning, T.Maniatis, E.F.Fritsch, J.Sambrook (1982). Les méthodes spécifiques des champignons sont décrites dans Sweigard et al.(Fungal Genetics Newletter. 44:52-53, 1997) pour les vecteurs de transformation fongiques utilisés, dans Orbach (Gene 150:159-162, 1994) pour la construction d'une banque cosmidique, dans Sweigard et al. (Fungal Genetics Newletter. 37:4-5,1990) pour la préparation d'ADN génomiques fongiques et dans Agnan et al. (Fungal Genetics and Biology 21 :292-301, 1997).

### Description des figures

**Figure 1: Autoradiogramme de l'Hybridation avec une sonde pAN7.1 du transfert sur** membrane de nylon de digestions de l'ADN génomique du mutant 763. (E:EcoRI; A: ApaI; C: ClaI; K: KpnI)
**Figure 2:** "Plasmid rescue" chez le mutant 763. ADN génomique du mutant 763 (en gras) avec site d'insertion du plasmide. Les positions des sites EcoRI et KpnI sur l'ADN génomique sont arbitraires.
**Figure 3: Locus d'insertion du plasmide pAN7.1 et fragment de restriction BglII-XhoI (6kb) complémentant la mutation** ***m763**.* La position de la sonde génomique (0,4 kb) issue de PRK763 est indiquée en gras. Les flèches indiquent la position des amorces de PCR pour l'amplification du point d'insertion du plasmide chez la souche sauvage. Le point d'insertion du plasmide pAN7.1 est également indiqué.
**Figure 4: Identification d'un domaine de "fermeture éclair à leucines" (leucin zipper)** basique. Consensus obtenu par alignement de la séquence de la protéine P763 avec celles des facteurs de transcription YAP-1 et GCN4 de *Saccharomyces cerevisiae* et MEAB *d'Aspergillus nidulans.* Ce domaine comprend un domaine basique (A) et un domaine "leucin zipper" proprement dit (B).
**Figure 5: Consensus obtenu par alignement de la séquence de la protéine 763 de *Magnaporthe grisea* avec celles des facteurs de transcription YAP-1 et GCN4 de *Saccharomyces cerevisiae* et CPC-1 de *Neurospora crassa.***
**Figure 6: Autoradiogrammes de l'hybridation avec une sonde de l'ADNc du gène 763 de membranes de Southern des produits d'amplification par RT-PCR et nested-PCR de l'ARNm de ce gène dans différentes conditions.**

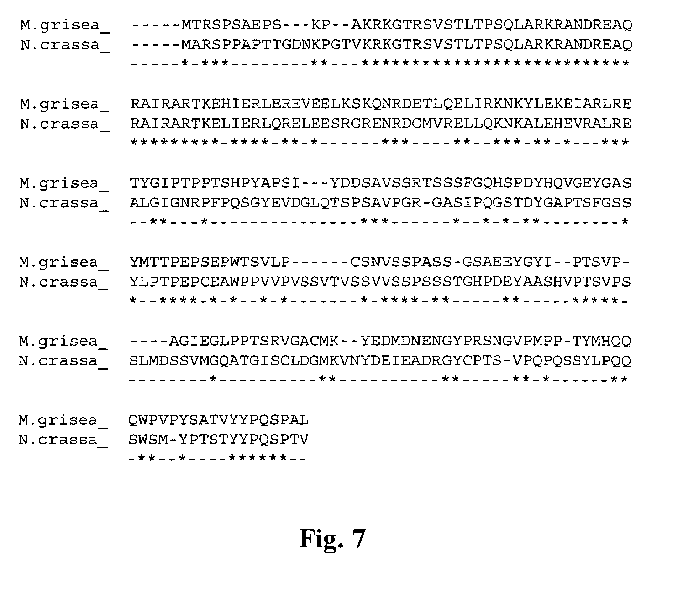

**Figure 7: Alignement de la protéine 763 de *Magnaporthe grisea* et de la protéine homologue de *Neurospora crassa.***
   Alignement réalisé à l'aide du programme clustal-W.
(* : acides aminés identiques)

### EXEMPLES

La stratégie employée pour parvenir à l'identification et la caractérisation du gène 763 essentiel à la pathogénie de *M. grisea* a comporté deux points principaux:
1) L'inactivation d'un gène essentiel à la pathogénie par l'insertion de manière aléatoire dans sa séquence nucléotidique d'un fragment d'ADN étranger (mutagenèse insertionnelle).
2) La récupération et la caractérisation de la séquence nucléotidique fongique ainsi modifiée, puis la démonstration de son implication dans la pathogénie du champignon vis-à-vis du riz et de l'orge.

Les étapes méthodologiques à franchir successivement ont été les suivantes:
1) L'obtention d'une collection d'isolats du champignon ayant intégré de manière aléatoire un fragment d'ADN étranger dans leur génome (transformants). En l'occurence, l'ADN étranger est un plasmide comportant le gène *hph* d'*Escherichia coli,* ce qui a permis leur sélection sur la base de la résistance à l'hygromycine. Il a été introduit dans le génome du champignon par transformation de protoplastes.
2) La recherche de transformants non pathogènes vis-à-vis du riz et de l'orge parmi la collection (mutants de pathogénie). Le critère retenu pour la non pathogénie d'un transformant a été l'incapacité à provoquer des lésions foliaires suite à l'inoculation de spores de ce transformant à des plants de riz et d'orge.
3) La démonstration génétique de l'inactivation d'un gène de pathogénie par le plasmide chez les mutants incapables d'infecter le riz et l'orge. Il s'agissait d'établir une liaison génétique complète entre le caractère de résistance à l'hygromycine, qui traduit la présence du plasmide dans le génome du mutant, et celui de la non pathogénie, qui traduit l'inactivation d'un gène essentiel à la capacité infectieuse du champignon. Ce degré de liaison a été évalué par l'analyse de la ségrégation des caractères de résistance à l'hygromycine et de non pathogénie chez les descendants d'un croisement entre le mutant étudié et une souche sauvage, pathogène vis-à-vis du riz et de l'orge et de signe sexuel compatible avec celui du mutant.
4) La récupération de la région génomique du champignon où s'est produite l'insertion du plasmide mutateur. Le principe a consisté à isoler un fragment d'ADN du mutant comportant à la fois des séquences plasmidiques et génomiques repérable grâce à une expérience d'hybridation avec une sonde d'origine plasmidique. La partie génomique incluse dans ce fragment a ensuite servi à isoler la région génomique sauvage complète selon le même principe.
5) La démonstration que la région génomique avoisinant le point d'insertion du plasmide contient le gène de pathogénie. Si le gène de pathogénie recherché se trouve dans la région génomique avoisinant le point d'insertion du plasmide, son introduction dans le génôme de l'isolat mutant, à l'aide d'un vecteur plasmidique comportant un autre marqueur de sélection, doit permettre de restaurer la pathogénie par complémentation de la fonction rendue déficiente par l'insertion du premier plasmide. La preuve en est donnée si les spores d'au moins un transformant obtenu par cette expérience sont capables de provoquer autant de lésions foliaires que la souche sauvage.
6) La caractérisation de la séquence génomique du champignon à proximité du point d'insertion du plasmide. Le produit du séquençage de la région génomique avoisinante au point d'insertion du plasmide est analysé avec des logiciels de traitement de séquences, de manière à tenter d'y mettre en évidence une séquence nucléotidique susceptible d'être traduite en séquence protéique (cadre de lecture ouvert). Cette recherche se fait sur la base de la recherche des signaux consensus d'initiation et de terminaison de la traduction en protéine. La preuve de l'existence d'un cadre de lecture ouvert (et donc d'un gène) dans cette région a été apportée par le clonage de l'unité transcriptionnelle correspondante, grâce au criblage d'une banque d'ADN complémentaires des ARN messagers (ADNc) avec une sonde produite à partir d'un fragment de cette région. La séquence de cet ADNc permet de déterminer avec précision la taille et la séquence primaire de la protéine correspondante, ainsi que la position d'éventuels introns dans la séquence génomique du gène.

### Exemple 1

### Mutagenèse insertionnelle

La transformation de protoplastes avec un plasmide intégratif portant un marqueur de sélection a été utilisée comme outil de mutagenèse insertionnelle afin de rechercher les gènes de pathogénie du champignon ascomycète et parasite du riz, *Magnaporthe grisea.* Les conditions de culture, d'obtention des protoplastes, de transformation ainsi que de purification et de stockage des transformants de *Magnaporthe grisea* sont décrites par Silué et al. (Physiol. Mol. Plant Pathol., 53, 239-251, 1998). La transformation a été réalisée avec 1 µg de plasmide pAN7.1 (Punt et al., Gene 78: 147-156, 1987) et 10⁷ protoplastes de la souche P1.2 de *M. grisea.* Cette souche provient de la collection du laboratoire de Phytopathologie du CIRAD de Montpellier. La sélection des transformants a été réalisée par incorporation d'hygromycine dans les milieux de culture gélosés, aux concentrations de 240 ppm pour le milieu de sélection primaire et de 120 ppm pour celui de sélection secondaire.

### Exemple 2

### Criblage de la collection de transformants et identification du mutant non pathogène 763

### A) Essais de pathogénie sur feuilles en survie

Les essais de pathogénie ont été réalisés sur deux variétés de riz, Maratelli et Sariceltick, et une variété d'orge, Express. Maratelli sont des variétés très sensibles à la pyriculariose et qui ne possèdent pas de gènes de résistance à la souche P1.2. Les variétés d'orge sont extrêmement sensibles à la pyriculariose. Le riz a été cultivé à 25°C le jour, 15°C la nuit avec une hygrométrie supérieure à 70%, l'orge en conditions froides (20-22°C). Des fragments de feuilles (2,5 cm) de riz et d'orge ont été prélevés dans la partie médiane de la plus jeune feuille de plants âgés d'une vingtaine de jours. Ces fragments ont été déposés dans des boîtes multicompartimentées contenant de l'eau gélosé à 1 % additionné de 2 mg / 1 de kinétine, milieu permettant leur survie durant 14 jours. Il est important de noter que le riz développe une forte résistance physiologique à la pyriculariose durant les périodes de fortes chaleurs. Cette résistance peut être atténuée par un apport de fertilisant azoté aux plants: deux arrosages avec une solution de sulfate d'ammonium 5g / m² à une semaine d'intervalle. Le deuxième arrosage a lieu 2 à 3 jours avant l'inoculation.

Les conditions de sporulation et de la préparation d'inoculum de spores de *M. grisea* sont décrites par Silué *et al.* (*op. cit.*). L'inoculation a été réalisée à l'aide d'un coton tige humide trempé dans une suspension de spores et passé sur les fragments de feuilles en survie. La quantité de spores déposée a été estimée en déposant une goutte de la suspension sur une lame de verre. Les symptômes ont été observés après 4-7 jours d'incubation à 24°C, 100 % d'hygrométrie. Chaque transformant a été testé sur quatre fragments de feuille de riz de chaque variété et quatre d'orge lors du criblage primaire. Le transformant 763 présente une réduction de la pathogénie quantifiée à 95 % du nombre de lésions causées par la souche sauvage. Le transformant 763 a été inoculé une seconde fois afin de confirmer son phénotype avec une suspension de spores de concentration ajustée à 10⁵ spores par ml. Les résultats illustrés danss le tableau ci-dessous.

**Tableau 1: Pénétration du mutant 763 dans les feuilles d'orge**

| Inoculation de feuilles d'orge par des gouttes de 35 microlitres contenant des spores (500 000 spores/ml) | |
|---|---|
| Exp 1 | 48h après inoculation, nombreux appressoria en surface, peu de pénétrations, quelques hyphes infectieux visibles, |
| | 6 jours, pas de lésion visible, brunissement au point de contact de la goutte |
| Exp 2 | 48h après inoculation, nombreux appressoria en surface, pénétration non observée 4 jours, pas de lésion visible, brunissement au point de contact de la goutte |
| Exp 3 | 48h après inoculation, nombreux appressoria en surface, peu de pénétrations, hyphes infectieux visibles dans la feuille, colonisation très ralentie par rapport à P12 |

### B) Essais de pathogénie sur plantes entières

De manière à confirmer le phénotype du mutant non pathogène 763 détecté par l'inoculation de feuilles en survie, le département de Phytopathologie du CIRAD de Montpellier a réalisé des inoculations de plantes entières avec les spores de ce mutant. Les deux cultivars de riz sensibles à la souche P 1.2, Maratelli et Sariceltick ont été semés et cultivés en serre. Trois applications d'azote ont été effectuées durant les trois premières semaines de culture (à 5, 10 et 20 jours après le semis). L'inoculation par pulvérisation d'une suspension de spores a lieu 10 à 15 jours après le dernier apport d'azote, selon le degré de maturité des plantes. La concentration en spores a été déterminée par comptage avec une cellule de Thoma et ajustée à une valeur 20000 spores/ml. Les suspensions de spores du mutant 763 et de la souche P1.2 non transformée ont été pulvérisées sur trente plantes à raison de 1 ml de suspension de spores par plante avec un aérographe. Une feuille de chacune de ces plantes a été collectée pour comptage du nombre de lésions après développement de celles-ci (5 à 7 jours). Une réduction de pathogénie de 93% a également été observée sur plantes entières (Voir tableau ci-dessous).

**Tableau 2: Pulvérisation sur plante entière (Riz, variété Sariceltik) d'une suspension de spores**

| | P12 (souche sauvage) | Mutant 763 | Diminution par rapport à P12 |
|---|---|---|---|
| Exp1 | | | |
| Spores | 42 lésions par feuille | 7 lésions par feuille | - 85 % |
| 25 000 sp/ml | taille des lésions: 3,5 mm2 | taille des lésions: 0,5 mm2 | -85 % |
| | | | |
| Exp 2 | | | |
| Spores | 30 lésions par feuilles | 2 lésions par feuille | - 93 % |
| 100 000 sp/ml | | | |

### Exemple 3

### Analyse phénotypique du mutant 763

Le mutant 763 est affecté d'une réduction de la pathogénie quantifiée à 93% du nombre de lésions causées par la souche sauvage, sans que sa capacité à sporuler ne soit amoindrie. De plus, si les rares lésions observées étaient bien visibles et formées d'une zone nécrotique entourée d'une marge brunâtre (symptôme typique de la pyriculariose), elles étaient toutes de petite taille et non sporulantes, contrairement à celles provoquées par la souche sauvage (-90% en surface). Un essai d'infection sur feuilles blessées montre que la progression des hyphes de ce mutant *in planta* reste limitée à la zone de blessure. L'analyse cytologique de l'infection chez ce mutant montre que le mutant parviens à pénétrer à travers la paroi des cellules épidermiques de l'orge, mais il est ensuite rapidement bloqué dans sa progression.

Le transformant 763 a une physiologie et une morphologie des conidies et du mycélium apparemment normales. Sa capacité à différencier des appressoria sur épiderme d'orge comme sur surfaces hydrophobes artificielles (PVC, Teflon, PET) n'est pas différente de celle de la souche sauvage.

Le croissance de ce mutant a également été étudiée en présence de sels et de drogues interférant avec l'assimilation de composés azotés. Il s'agissait de voir s'il présentait le phénotype de perte de la répression métabolique de l'azote, caractéristique du mutant *meaB* d'*Aspergillus nidulans* (voir plus loin l'analyse moléculaire; Polley et Caddick, 1996). Chez ce champignon, la répression métabolique de l'azote se traduit en présence d'ammonium ou de L-glutamine par l'inhibition des gènes nécessaires au prélèvement et à l'utilisation d'autres sources d'azote. La mutation *meaB* est caractérisée par sa résistance au méthylammonium (un inducteur toxique de la répression métabolique de l'azote) mais également par sa résistance à la parafluorophénylalanine et par son hypersensibilité à la toxicité nitrique. Le mutant 763 n'a pas un phénotype différent de celui de la souche sauvage dans toutes les conditions testées. 763 croît également normalement sur milieu minimum (MM).

### Exemple 4

### Analyse génétique et moléculaire du mutant 763

20 ascospores en vrac et une tétrade issues du croisement M4 X 763 ont été analysées. Les résultats de cette analyse figurent dans le tableau ci-dessous et montrent que la résistance à l'hygromycine coségrège avec la perte du pouvoir pathogène: le gène de pathogénie muté est étiqueté par le plasmide pAN7.1 chez 763.

**Tableau 3: Analyse des descendants du croisement M4x763**

| **Tetrade parentale** | | |
|---|---|---|
| **Ascospore** | **Hyg.** | **Path.** |
| 1 | s | + |
| 2 | s | + |
| 3 | s | + |
| 4 | s | + |
| 5 | R | - |
| 6 | R | - |
| 7 | R | - |
| 8 | N.D. | N.D. |

| **Ascospores en vrac** | | |
|---|---|---|
| **Ascospore** | **Hyg.** | **Path.** |
| 1 | s | + |
| 2 | N.D. | N.D. |
| 3 | R | - |
| 4 | R | - |
| 5 | R | - |
| 6 | R | - |
| 7 | R | - |
| 8 | S | + |
| 9 | R | - |
| 10 | R | - |
| 11 | R | - |
| 12 | s | + |
| 13 | s | + |
| 14 | s | + |
| 15 | s | + |
| 16 | R | - |
| 17 | R | - |
| 18 | R | - |
| 19 | s | + |
| 20 | s | + |

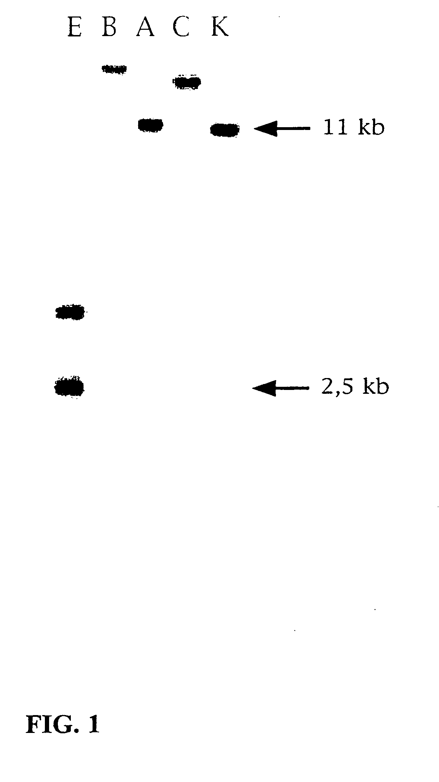

Le nombre de copies du plasmide pAN7.1 présentes dans le génôme de ce transformant et la position relative du point d'intégration ont été déterminés par hybridation avec une sonde plasmidique (voir Figure 1). Trois types d'enzymes de restriction ont été employées en fonction du nombre de coupures voulues: *Eco*RI (2 coupures); *Bam*HI (1 coupure); *Apa*I, *Cla*I et *Kpn*I (aucune coupure). Les profils d'hybridation des fragments de restriction obtenus montrent que ce transformant ne comporte qu'une seule copie du plasmide (3 fragments *Eco*RI, un seul fragment pour *Bam*HI, *Apa*I, *Cla*I et *Kpn*I). Par ailleurs, l'unique fragment d'hybridation *Bam*HI est de taille supérieure à 6,75 kb. Ceci indique que la copie du plasmide intégrée dans le génôme de ce transformant ne possède pas à ces extremités les deux sites *Bam*HI attendus suite à une transformation en présence cet enzyme de restriction. Selon l'analyse faite sur de nombreux transformants issus de transformations REMI, il est probable que l'intégration du plasmide ait entrainé de courtes délétions au niveau de l'une ou des deux extrémités cohésives du site *Bam*HI plasmidique, ne créant ainsi aucun site de restriction *Bam*HI au niveau des jonctions entre l'ADN plasmidique et l'ADN génomique du transformant. En ce qui concerne les digestions avec les enzymes ne coupant pas dans la séquence de pAN7.1 (*Apa*I, *Cla*I et *Kpn*I), le fragment de restriction le plus petit contenant la totalité du plasmide a été obtenu dans la voie correspondante à la digestion *Kpn*I (taille de 11 kb). Une expérience complémentaire d'hybridation de fragments de restrictions *Ssp*I de l'ADN génomique du transformant 763 par une sonde pUC19 a pu montrer que les séquences de l'origine de réplication du plasmide dans *E. coli* et du gène de résistance à l'ampicilline étaient intactes.

### Exemple 5

### Clonage et caractérisation du gène de pathogénie 763

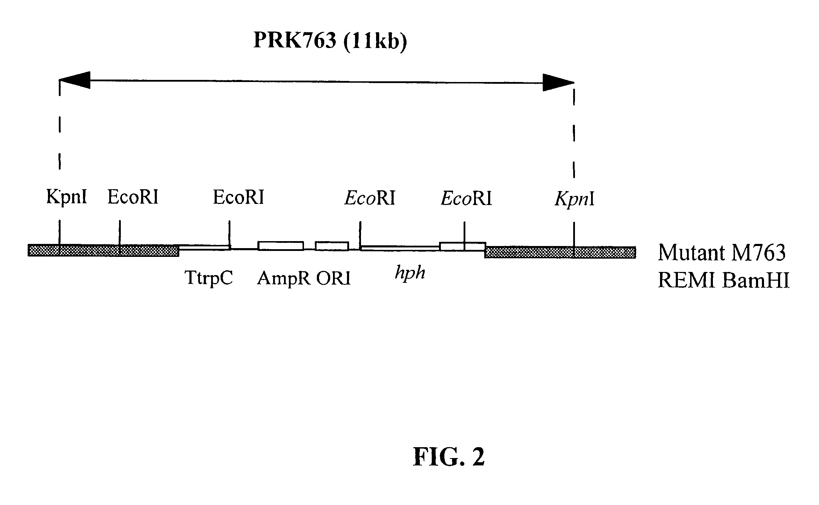

La technique du "plasmid rescue" (Timberlake, 1991) a été employée pour cloner les régions génomiques situées au point d'insertion du plasmide. De par sa petite taille, c'est le fragment *Kpn*I de 11 kb identifié lors de l'analyse moléculaire du mutant qui a été choisi pour réaliser cette expérience (Figure 2). 4 colonies résistantes à l'ampicilline obtenues ont fait l'objet d'une analyse par restriction de leur ADN plasmidique. Une colonie portant le plasmide attendu (PRK763) a été striée et multipliée en vue d'une maxipréparation d'ADN. Un fragment d'ADN génomique *Nde*I*-Ssp*I de PRK763 d'une taille de 0,4 kb, répéré suite au séquençage des régions d'origine génomique de ce plasmide, a été utilisé pour sonder la banque cosmidique de la souche 96/0/76. Deux cosmides hybridant avec ce fragment ont été isolés (21 C7 et 35F3).

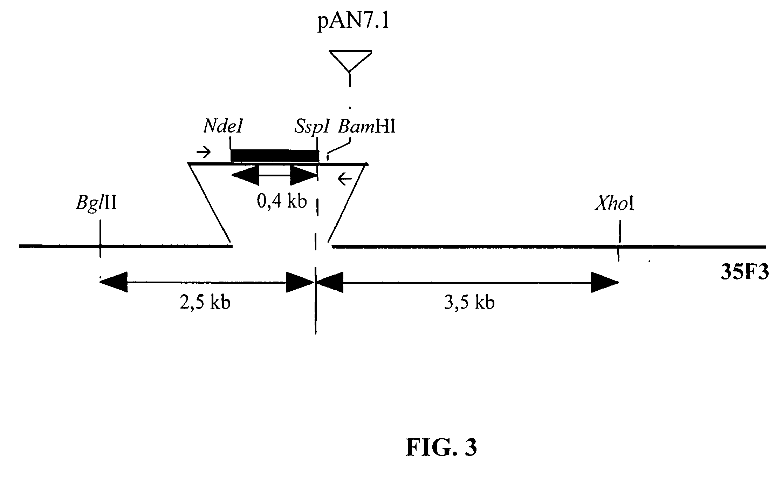

Un fragment de restriction *Xho*I*-Bgl*II de 6kb du cosmide 35F3 hybridant avec le fragment de restriction *Nde*I*-Ssp*I de PRK763 a été cloné dans le plasmide pCB1265 (Figure 3). Cette construction appelée pC763 a été introduite par transformation de protoplastes dans le génôme du mutant 763. Un essai de pathogénie sur feuilles détachées a montré que les transformants résistants à la phosphinothricine obtenus ont le même degré de virulence que la souche sauvage.

La banque d'ADN complémentaires des ARN messagers de gènes exprimés dans une culture en milieu complet liquide a été criblée avec le fragment *Nde*I*-Ssp*I de PRK763. Deux types de clones longs d'environ 2 kb été récupérés. L'un était plus court que l'autre de 113 pb en son extrémité 5', mais plus long de 16 pb en son extrémité 3' et ce juste avant la séquence polyadénylée terminale. Cette séquence polyadénylée était présente aux extrémités 3' des deux types de clones isolés. La comparaison de cette séquence d'ADNc avec celle de l'ADN génomique sauvage correspondant a permis de mettre en évidence 3 introns, de respectivement 153, 78 et 108 pb. Les positions des signaux d'initiation et de terminaison de la traduction dans la séquence de l'ADNc définissent un cadre ouvert de lecture long de 714 pb. Il débute à 25 pb de l'extrémité 5' de la séquence du clone d'ADNc le plus long et se termine à 1,22 kb de l'extrémité 3' de la séquence de ce même clone. Cette longue séquence 3'-terminale non traduite comporte de nombreux signaux de terminaisons potentiels dans les trois phases de lectures possibles.

La recherche de protéines de séquences homologues à celle de P763 a été réalisée avec le programme d'alignement de séquences BLASTP 2.0.8 (Altschul *et al.,* 1997) dans toutes les bases de données disponibles en utilisant les paramètres par défaut. Les seules protéines présentant un degré d'homologie significatif avec la protéine de pathogénie 763 de *Magnaporthe grisea* sont le facteur de transcription putatif MEAB d'*Aspergillus nidulans* ainsi que les facteurs de transcriptions GCN4 et YAP1 de *Saccharomyces cerevisiae.*

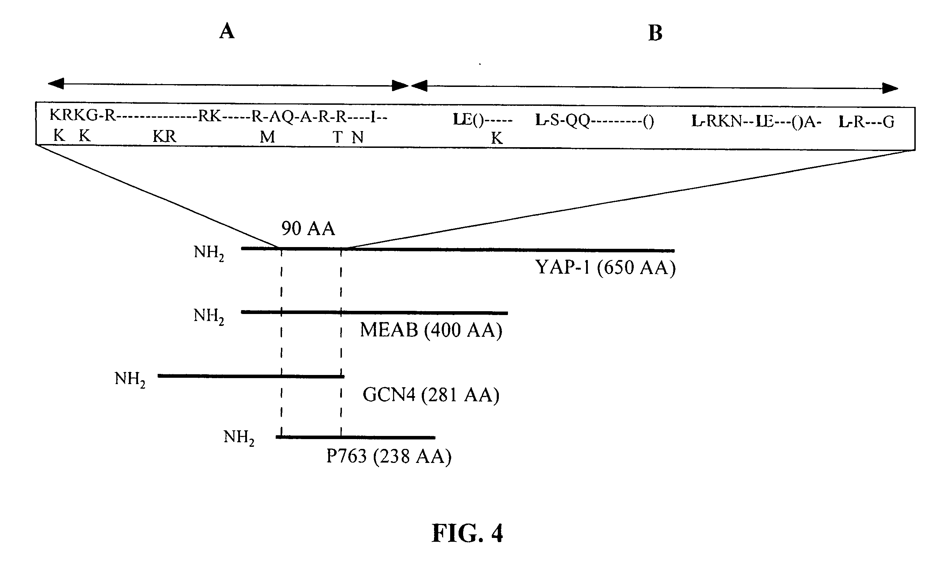

MEAB serait impliquée dans le contrôle de l'assimilation de l'azote en fonction de la nature des sources disponibles de cet élément (Polley et Caddick, FEBS letters 388:200-205, 1996). De par sa séquence, MEAB est apparentée à la famille des facteurs de transcription eucaryotes de type bZIP, composé d'un motif à dominante basique de liaison séquence-spécifique à l'ADN suivi d'un autre dit "à fermeture-éclair à leucines", nécessaire à la dimérisation de la protéine. Le degré de similarité entre P763 et MEAB est maximal dans la partie amino-terminale de leurs séquences, celle correspondant au domaine bZIP. Cette région mise à part, la séquence de MEAB est plus longue (400 AA contre 238 chez P763) et peu ressemblante à celle de P763 dans sa partie carboxy-terminale (Figure 4).

**Tableau 4: Recherche de protéines homologues de la protéine déduite du gène 763 (Blast P version 2.0.8)**

| Score E | % d'identité et d'homologie au niveau du domaine b-ZIP (63 acides aminés) | |
|---|---|---|
| 0.00009 | 38 % et 54 % | MeaB facteur de transcription putatif d'A. nidulans avec b-ZIP |
| 0.002 | 31 % et 55 % | YAP1, facteur de transcription de S. cerevisae avec b-ZIP |
| 0.002 | 40 % et 55 % | GCN4, facteur de transcription de S. cerevisae avec b-ZIP |

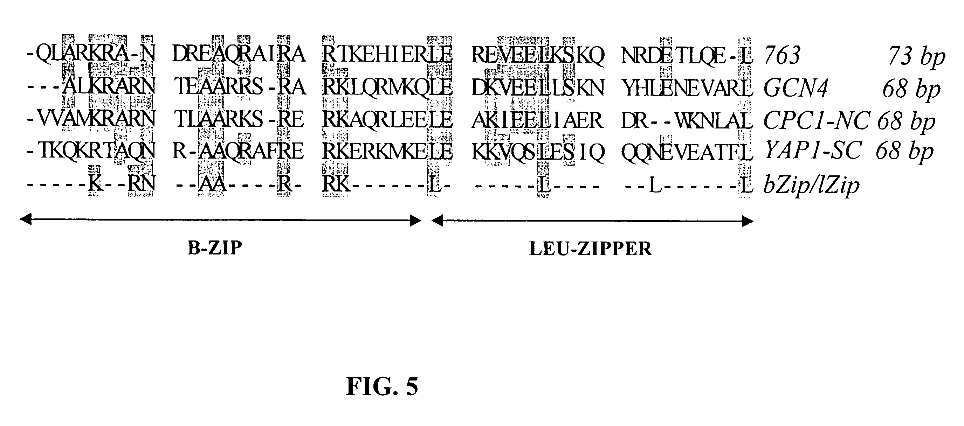

L'analyse phénotypique du mutant 763 en fonction de son comportement vis-à-vis de plusieurs drogues interférant avec le métabolisme de l'azote laisse penser que le gène étiqueté par le plasmide chez le mutant 763 ne serait pas l'équivalent de *MEAB* chez *Magnaporthe grisea.* La séquence du domaine bZIP de P763 s'aligne partiellement dans la même recherche avec celles de deux facteurs de transcription de *Saccharomyces cerevisiae,* GCN4 (protéine de régulation de l'expression de gènes de la biosynthèse des acides aminés; Hinnebusch, PNAS 81:6442-6446, 1984) et YAP1 (activateur de la transcription de gènes de défense cellulaire contre le stress oxydatif; Schnell *et al.,* Curr. Genet. 21 (4-5):269-731992) et avec le gène CPC-1 de *Neurospora crassa* (figure 5). Les gènes de séquence homologue à celle du gène de GCN4 ont été identifiés chez les champignons filamenteux *Neurospora crassa* (Paluh *et al.,* PNAS 85 (11) 3728-3732, 1988) avec le gène CPC-1 et *Cryphonectria parasitica* (Wang *et al.,* Fungal Genet. Biol. 23(1):81-94, 1998) et sont différents du gène 763 bien qu'apparentés.

### Exemple 6

### Expression du gène de pathogénie 763

Un Northern blot réalisé avec 10 µg d'ARN totaux extraits d'échantillons de mycélium cultivé dans plusieurs conditions (culture liquide en milieu complet ou en milieu minimum) a été hybridé sans succès avec une sonde correspondant à la séquence de l'ADNc du gène 763.

Une expérience de RT-PCR a été réalisée avec des amorces situées de part et d'autre du signal de terminaison de la traduction putatif (défini grâce de l'analyse de la séquence de l'ADNc) et 5 µg d'ARN totaux extrait de mycélium d'une culture liquide en milieu complet. Le produit d'amplification, détecté par hybridation avec une sonde 763, a été cloné et séquencé. Il ne présente pas de différences de taille ou de séquence avec les clones d'ADNc isolés auparavant, notamment dans la partie correspondante à la séquence 3' non traduite de l'ARN messager du gène.

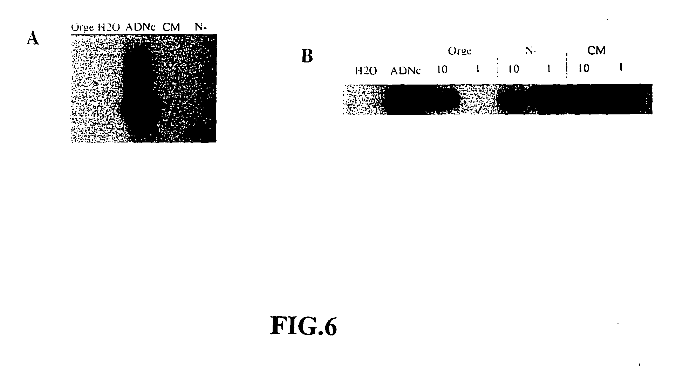

Une expérience de nested RT-PCR a été réalisée avec 5 µg d'ARN de feuilles d'orge infectées extraits 20 heures après inoculation et une amplification secondaire avec un deuxième couple d'amorces interne. Un produit d'amplification a été détecté par hybridation avec une sonde 763, mettant en évidence l'expression de ce gène au cours des étapes précoces de la colonisation de l'hôte **non humain** (Figure 6).

## Revendications

1. Polynucléotide **caractérisé en ce qu'**il comprend un polynucléotide choisi parmi les polunucléotides suivants:
a) le polynucléotide de la SEQ ID No.1; et
b) le polynucléotide de la SEQ ID No.2; et
c) le polynucléotide dont la séquence est comprise entre la position 17 et la position 733 de la SEQ ID No. 2

2. Polypeptide **caractérisé en ce qu'**il comprend le polypeptide 763 de la SEQ ID No. 3 ou un fragment biologiquement actif du polypeptide 763 de la SEQ ID No. 3 ayant une activité de facteur de transcription fonctionnel dans les champignons.

3. Polypeptide **caractérisé en ce qu'**il comprend un polypeptide homologue à au moins 80% au polypeptide 763 de la SEQ ID No.3.

4. Polypeptide selon la revendication 3 **caractérisé en ce qu'**il comprend un facteur de transcription fonctionnel dans les champignons.

5. Cassette d'expression **caractérisée en ce qu'**elle comprend dans le sens de la transcription:
a) un promoteur fonctionnel dans un organisme hôte non humain ; et
b) un polynucléotide selon la revendication 1 et
c) une séquence terminatrice dans ledit organisme hôte non humain.

6. Cassette d'expression **caractérisée en ce qu'**elle comprend dans le sens de la transcription:
a) un promoteur fonctionnel dans un organisme hôte non humain; et
b) un polynucléotide codant pour un polypeptide selon l'une des revendications 2-4; et
c) une séquence terminatrice dans ledit organisme hôte non humain.

7. Vecteur comprenant un polynucléotide selon la revendication 1 ou une cassette d'expression selon l'une des revendications 5 ou 6.

8. Organisme hôte non humain transformé avec un polynucléotide selon la revendication 1 ou une cassette d'expression selon l'une des revendications 5 ou 6 ou un vecteur selon la revendication 7.

9. Procédé de transformation des organismes hôtes non humains par intégration dans ledit organisme hôte d'au moins un polynucléotide selon la revendication 1 ou une cassette d'expression selon l'une des revendications 5 ou 6 ou un vecteur selon la revendication 7.

10. Procédé pour l'identification de composés inhibant la pathogénie des champignons comprenant les étapes suivantes:
a) mettre en contact ledit composé avec un polypeptide 763 selon l'une des revendications 2-4; et
b) détecter la fixation dudit composé audit polypeptide.

11. Procédé pour l'identification de composés inhibant la pathogénie des champignons comprenant les étapes suivantes:
a) mettre en contact ledit composé avec un organisme hôte non humain transformé avec un polynucléotide un vecteur selon l'une des revendication 1 ou 7, ledit organisme hôte exprimant un gène rapporteur sous le contrôle du promoteur du gène 763; et
b) détecter l'inhibition de l'expression dudit gène rapporteur.

12. Procédé pour l'identification de composés inhibant la pathogénie des champignons comprenant les étapes suivantes:
a) mettre en contact ledit composé avec un organisme hôte non humain transformé un polynucléotide selon la revendication 1 ou une cassette d'expression selon l'une des revendications 5 ou 6 ou un vecteur selon la revendication 7, ledit organisme hôte exprimant un polypeptide 763; et
b) détecter l'inhibition de l'expression dudit polypeptide 763.

13. Procédé selon l'une des revendications 10-12 comprenant en outre une étape dans laquelle on détermine si ledit composé inhibe la pathogénie des champignons.

14. Utilisation d'un polynucléotide, d'une cassette d'expression, d'un vecteur, d'un organisme hôte non humain ou/et du polypeptide 763 selon l'une des revendications 1-8, pour l'identification des gènes impliqués dans la pathogénie des champignons ou pour l'identification de nouvelles molécules fongicides inhibitrices de la pathogénie des champignons.

## Claims

1. Polynucleotide, **characterized in that** it comprises a polynucleotide chosen from the following polynucleotides:
a) the polynucleotide of SEQ ID No. 1; and
b) the polynucleotide of SEQ ID No. 2; and
c) the polynucleotide, the sequence of which is included between position 17 and position 733 of SEQ ID No. 2.

2. Polypeptide, **characterized in that** it comprises the polypeptide 763 of SEQ ID No. 3 or a biologically active fragment of the polypeptide 763 of SEQ ID No. 3 having the activity of a transcription factor which is functional in fungi.

3. Polypeptide, **characterized in that** it comprises a polypeptide at least 80% homologous to the polypeptide 763 of SEQ ID No. 3.

4. Polypeptide according to Claim 3, **characterized in that** it comprises a transcription factor which is functional in fungi.

5. Expression cassette, **characterized in that** it comprises, in the direction of transcription:
a) a promoter which is functional in a non human host organism; and
b) a polynucleotide according to Claim 1; and
c) a sequence which is a terminator sequence in said non human host organism.

6. Expression cassette, **characterized in that** it comprises, in the direction of transcription:
a) a promoter which is functional in a non human host organism; and
b) a polynucleotide encoding a polypeptide according to one of Claims 2-4; and
c) a sequence which is a terminator sequence in said non human host organism.

7. Vector comprising a polynucleotide according to Claim 1 or an expression cassette according to either of Claims 5 and 6.

8. Non human host organism transformed with a polynucleotide according to Claim 1 or an expression cassette according to either of Claims 5 and 6 or a vector according to Claim 7.

9. Method for transforming non human host organisms by integrating into said host organism at least one polynucleotide according to Claim 1 or an expression cassette according to either of Claims 5 and 6 or a vector according to Claim 7.

10. Method for identifying compounds which inhibit fungal pathogenesis, comprising the following steps:
a) bringing said compound into contact with a polypeptide 763 according to one of Claims 2-4; and
b) detecting the binding of said compound to said polypeptide.

11. Method for identifying compounds which inhibit fungal pathogenesis, comprising the following steps:
a) bringing said compound into contact with a non human host organism transformed with a polynucleotide or a vector according to either of Claims 1 and 7, said host organism expressing a reporter gene under the control of the promoter of gene 763; and
b) detecting the inhibition of the expression of said reporter gene.

12. Method for identifying compounds which inhibit fungal pathogenesis, comprising the following steps:
a) bringing said compound into contact with a non human host organism transformed with a polynucleotide according to Claim 1 or an expression cassette according to either of Claims 5 and 6 or a vector according to Claim 7, said host organism expressing a polypeptide 763; and
b) detecting the inhibition of the expression of said polypeptide 763.

13. Method according to one of claims 10-12, also comprising a step in which it is determined whether said compound inhibits fungal pathogenesis.

14. Use of a polynucleotide, of an expression cassette, of a vector, of a non human host organism and/or of the polypeptide 763 according to one of Claims 1-8, for identifying genes involved in fungal pathogenesis or for identifying novel fungicidal molecules which inhibit fungal pathogenesis.

## Patentansprüche

1. Polynukleotid, **dadurch gekennzeichnet, daß** es ein Polynukleotid aus der Reihe der folgenden Polynukleotide enthält:
a) Polynukleotid gemäß SEQ ID No. 1; und
b) Polynukleotid gemäß SEQ ID No. 2; und
c) Polynukleotid mit der Sequenz zwischen Position 17 und Position 733 gemäß SEQ ID No. 2.

2. Polypeptid, **dadurch gekennzeichnet, daß** es das Polypeptid 763 gemäß SEQ ID No. 3 oder ein biochemisch aktives Fragment des Polypeptids 763 gemäß SEQ ID No. 3 mit einer in Pilzen funktionellen Transkriptionsfaktoraktivität enthält.

3. Polypeptid, **dadurch gekennzeichnet, daß** es ein Polypeptid mit mindestens 80% Homologie zu dem Polypeptid 763 gemäß SEQ ID No. 3 enthält.

4. Polypeptid nach Anspruch 3, **dadurch gekennzeichnet, daß** es einen in Pilzen funktionellen Transkriptionsfaktor enthält.

5. Expressionskassette, **dadurch gekennzeichnet, daß** sie in Transkriptionsrichtung
a) einen in einem nichtmenschlichen Wirtsorganismus funktionellen Promoter; und
b) ein Polynukleotid nach Anspruch 1 und
c) eine Terminatorsequenz in dem nichtmenschlichen Wirtsorganismus
enthält.

6. Expressionskassette, **dadurch gekennzeichnet, daß** sie in Transkriptionsrichtung
a) einen in einem nichtmenschlichen Wirtsorganismus funktionellen Promoter; und
b) ein Polynukleotid, das für ein Polypeptid nach einem der Ansprüche 2-4 codiert; und
c) eine Terminatorsequenz in dem nichtmenschlichen Wirtsorganismus
enthält.

7. Vektor, enthaltend ein Polynukleotid nach Anspruch 1 oder eine Expressionskassette nach einem der Ansprüche 5 oder 6.

8. Nichtmenschlicher Wirtsorganismus, der mit einem Polynukleotid nach Anspruch 1 oder mit einer Expressionskassette nach einem der Ansprüche 5 oder 6 oder mit einem Vektor nach Anspruch 7 transformiert ist.

9. Verfahren zur Transformation von nichtmenschlichen Wirtsorganismen durch Integration von mindestens einem Polynukleotid nach Anspruch 1 oder einer Expressionskassette nach einem der Ansprüche 5 oder 6 oder einem Vektor nach Anspruch 7 in diesen Wirtsorganismus.

10. Verfahren zur Identifikation von Verbindungen, die die Pathogenität von Pilzen hemmen, wobei das Verfahren die folgenden Schritte umfaßt:
a) In-Kontakt-Bringen der Verbindung mit einem Polypeptid 763 nach einem der Ansprüche 2-4; und
b) Nachweisen der Bindung dieser Verbindung an das Polypeptid.

11. Verfahren zur Identifikation von Verbindungen, die die Pathogenität von Pilzen hemmen, wobei das Verfahren die folgenden Schritte umfaßt:
a) In-Kontakt-Bringen der Verbindung mit einem nichtmenschlichen Wirtsorganismus, der mit einem Polynukleotid einem Vektor nach einem der Ansprüche 1 oder 7 transformiert ist, wobei der Wirtsorganismus ein Reportergen unter der Kontrolle des Promoters von Gen 763 exprimiert; und
b) Nachweisen der Hemmung der Expression des Reportergens.

12. Verfahren zur Identifikation von Verbindungen, die die Pathogenität von Pilzen hemmen, wobei das Verfahren die folgenden Schritte umfaßt:
a) In-Kontakt-Bringen der Verbindung mit einem nichtmenschlichen Wirtsorganismus, der mit einem Polynukleotid nach Anspruch 1 oder einer Expressionskassette nach einem der Ansprüche 5 oder 6 oder einem Vektor nach Anspruch 7 transformiert ist, wobei der Wirtsorganismus ein Polypeptid 763 exprimiert; und
b) Nachweisen der Hemmung der Expression des Polypeptids 763.

13. Verfahren nach einem der Ansprüche 10-12, das weiterhin einen Schritt umfaßt, bei dem man bestimmt, ob die Verbindung die Pathogenität von Pilzen hemmt.

14. Verwendung eines Polynukleotids, einer Expressionskassette, eines Vektors, eines nichtmenschlichen Wirtsorganismus und/oder von Polypeptid 763 nach einem der Ansprüche 1-8 zum Identifizieren von Genen, die in der Pathogenität von Pilzen impliziert sind oder zum Identifizieren von neuen fungiziden Molekülen, die die Pathogenität von Pilzen hemmen.
